# EUROPEAN PATENT APPLICATION

(11) **EP 4 834 421 A1**
(43) Date of publication of application: **04.11.2026**
(21) Application number: 25173539.5
(22) Date of filing: 30.04.2025
(51) Int. Cl.: A61N 2/02

(54) **DEVICE AND METHOD FOR MAGNETIC FIELD EXPOSURE**

(71) Applicant: Helmholtz-Zentrum Dresden - Rossendorf e.V., 01328 Dresden (DE)
(72) Inventor: HERRMANNSDÖRFER, Thomas, 01328 Dresden (DE); FUNK, Richard, 01328 Dresden (DE); PAL, Arun, 01328 Dresden (DE)
(74) Representative: Gruner, Leopold Joachim

(57) **Abstract**

The present invention pertains to an apparatus configured to expose a target to pulsed and/or oscillating magnetic fields, wherein the target comprises neurons, in particular motor neurons, which is further configured for the treatment of subjects suffering from or at risk of neurodegenerative diseases. In addition, the invention comprises a method for increasing the rate of organelle transport in motor neurons in vitro by transient magnetic field exposure, and a control and supply circuit designed to generate the required magnetic field.

## Description

### TECHNICAL FIELD

The present invention relates to the field of magnetic field generation devices, in particular to an apparatus configured to expose a target to pulsed and/or oscillating in addition to static magnetic fields, wherein the target comprises neurons, in particular motor neurons, which is further configured for the treatment of subjects suffering from or at risk of neurodegenerative diseases. In addition, the invention comprises a method for increasing the rate of organelle transport in motor neurons in vitro by transient magnetic field exposure, and a control and supply circuit designed to generate the required magnetic field.

### STATE OF THE ART

Neurodegenerative diseases such as Alzheimer's, Parkinson's, Huntington's, Creutzfeldt-Jakob Disease (CJD) and the very common diabetic polyneuropathy have in common that their underlying progressive course cannot yet be stopped, let alone reversed. The best that can be done is to relieve the symptoms, mainly through medication and occupational therapy, and to slow down the underlying degeneration of the affected nerve cells. In the case of Parkinson's disease, which is widespread, symptomatic relief of motor deficits with drugs is successful in the early stages but becomes increasingly difficult in later stages.

In particular motor neuron diseases, such as amyotrophic lateral sclerosis (ALS), (also referred to as motor neuron disease (MND) or, in some regions, Lou Gehrig's disease), are among the most severe and currently untreatable conditions. Presently available pharmacological treatments - such as riluzole and edaravone - have been shown to only modestly slow disease progression, typically by a matter of months, and do not address the underlying cellular dysfunctions. One notable pathological characteristic in ALS is the impairment of axonal transport, which manifests as a reduction in the speed of organelle transport within motor neurons. This impairment is believed to contribute to synaptic dysfunction and the eventual loss of motor neuron viability. The degeneration of the axons of spinal motoneurons results in progressive muscle paralysis, leading to death within three to five years after diagnosis on average. Other examples of motor neuron diseases include primary lateral sclerosis (PLS), which mainly affects upper motor neurons; progressive muscular atrophy (PMA), which mainly affects lower motor neurons; and spinal muscular atrophy (SMA), which affects motor neurons in the spinal cord, leading to progressive muscle weakness.

An alternative to drug therapies may be presented by non-invasive neuromodulation techniques, such as transcranial magnetic stimulation (TMS) and electrical muscle stimulation (EMS), which have been clinically evaluated for various neurological conditions. TMS is based on the physical principle of electromagnetic induction. An electromagnetic coil of low inductance aligned tangentially to the skull generates a brief magnetic field of 200 to 600 µs duration, i.e. electromagnetic frequencies in the range of 2 kHz to 5 kHz. Magnetic flux densities of up to 3 Tesla are used. Currently known TMS magnetic systems are therefore unable to address neurodegenerative diseases in the spinal cord and spinal motor or sensory periphery as a whole. Although these modalities have demonstrated some benefit in terms of neurophysiological modulation, the evidence for therapeutic effects based solely on the application of alternating or modulated magnetic fields remains limited Several devices have been proposed to apply such magnetic fields for a broad spectrum of clinical indications, including motor neuron diseases. However, none have yet provided conclusive, evidence-based demonstrations of the direct influence of modulated magnetic fields on cellular processes, such as the enhancement of organelle transport in motor neurons.

Further current diagnostic and therapeutic applications of magnetic field-based devices include magnetic resonance imaging (MRI), but evidence-based therapeutic devices for neuronal diseases have not yet been demonstrated. Coil systems for medical diagnostics (e.g. magnetic resonance imaging, MRI) use static fields in the range of 1 Tesla to about 10 Tesla (T). MRI exploits the property of certain atomic nuclei to absorb radio frequency (RF) energy when placed in a strong external magnetic field (1 to 10 T); the resulting spin polarization can induce an RF signal in a radio frequency coil and thus be detected. In other words, the nuclear magnetic spin of the protons in the hydrogen nuclei resonates with the incident RF waves and emits coherent radiation with compact direction, energy (frequency) and phase, which is easily detected by RF antennas close to the subject under investigation.

There are several scientific publications investigating the potential of non-ionizing radiation, in particular static or alternating electromagnetic fields, under various conditions, but so far no one has been able to produce a set of conditions directly linked to an effect at the cellular or organism level in the treatment of neurodegenerative diseases. The electromagnetic fields studied range from static electric or magnetic fields (SEF/SMF), through low and intermediate frequency electromagnetic fields (1 to 100 kHz), to radiofrequency fields (100 kHz to 30 THz), a frequency range of 12 orders of magnitude. A general overview of approaches in the 0 to 10 THz range is reported by Mattsson and Simkó (Emerging medical applications based on non-ionizing electromagnetic fields from 0 Hz to 10 THz. Med Devices (Auckl) 2019, 12:347-68. Doi: 10.2147/MDER.S214152), another summary is reported by Mansourian M, Shanei A (Evaluation of pulsed electromagnetic field effects: a systematic review and meta-analysis on highlights of two decades of research in vitro studies. Biomed Res Int. 2021; 2021:6647497. doi:10.1155/2021/6647497).

Recent advances in cellular and molecular effects of magnetic fields have been reported by Tota et al. (J. Cellular and Molecular Effects of Magnetic Fields. Int. J. Mol. Sci. 2024, 25, 8973, DOI: 10.3390/ijms25168973). System level effect studies of low-frequency electromagnetic fields were summarized by Tian et al. (System-level biological effects of extremely low-frequency electromagnetic fields: an in vivo experimental review. Front Neurosci. 2023 Oct 6;17. doi:10.3389/fnins.2023.1247021). There are existing publications showing altered gene expression (Cook and Petrucelli; Genetic Convergence Brings Clarity to the Enigmatic Red Line in ALS. Neuron 2019, 101, 1057-1069, doi:10.1016/j.neuron.2019.02.032) and signaling pathways (Tumm et al., Induction of CAMP-Dependent Protein Kinase A Activity in Human Skin Fibroblasts and Rat Osteoblasts by Extremely Low-Frequency Electromagnetic Fields. Radiat Environ Biophys 1999, 38, doi:10.1007/s004110050155; Nie and Henderson, MAP Kinase Activation in Cells Exposed to a 60 Hz Electromagnetic Field. J Cell Biochem 2003, 90, 1197-1206, doi:10.1002/jcb.10704) in human cells through magnetic stimulation.

Many magnetic exposure and therapeutic devices have been disclosed in the state of the art, but there remains a serious lack of evidence-based data correlating magnetic field exposure with a measurable therapeutic or cellular effect. Furthermore, despite the wide range of devices that have been disclosed, none have been approved by regulatory agencies such as the EMA or the FDA for use as a therapeutic option, particularly in the treatment of neurodegenerative diseases such as amyotrophic lateral sclerosis (ALS).

In preferred embodiments, a number of medical devices for mixed uses are known, each comprising at least one magnetic field generation device, such as coil electromagnets. For example, patent specification US 8758216 B2 discloses devices and methods for magnetic field therapy aimed at increasing bone density. The disclosed method involves applying external electromagnetic fields to body tissues using a distributor comprising a plurality of coils, with output field strengths of 1 to 100 µT and repetition frequencies ranging from 1 to 100 Hz. However, the specification does not disclose the magnetic field strength at the actual application site, thereby limiting its therapeutic relevance.

Similarly, the document ITFO20130007A1 discloses a device with a specific pulse program that generates a magnetic field with amplitudes between 0.5 mT and 8 mT, yet it does not address applications in neural degenerative diseases. Regarding muscle activation, patent specification WO 2002022205 A1 teaches a method of exercising muscles during sleep using an implanted microstimulator in combination with a plurality of inductive coils integrated into a mattress, along with a controller for generating power and control signals. In another embodiment, patent specification WO 2022122923 A1 discloses an actively cooled applicator for magnetic therapy that is configured to deliver magnetic fields of sufficient intensity, frequency, and repetition to induce supramaximal muscle contraction. Furthermore, application US 10653881 B2 **(**WO 2018132678 A1**)** describes a system for tissue engineering using pulsed electromagnetic fields (PEMF), which improves musculoskeletal tissue stimulation via an electromagnetic field transmitter generating dual pulse repetition frequencies-one between 5 kHz and 50 kHz and another between 2 kHz and 6 kHz.

In another example, document WO 2011076395 A1 discloses a magnetic field treatment apparatus comprising a horizontally disposed base (configured as a couch) with at least one coil embedded in or beneath the base, and additional coils extending vertically from opposing side parts to produce nuclear magnetic resonances. This disclosure, however, does not relate the apparatus to a specific therapeutic use.

Some documents address the stimulation of microcirculation by applying modulated magnetic fields to tissue. For instance, patent specification US 6786859 B2 discloses an apparatus comprising a signal generation unit that produces an electrical treatment signal with superimposed frequency components (approximately 300 Hz, 600 Hz, 800 Hz, and 1,000 Hz) and an induction coil mat. Additionally, another application discloses a system for applying low-frequency magnetic fields to biological tissues, where a field strength between 0.01 mT and 5 mT is achieved at a depth of 1 cm, and the oscillating electrical signal has a frequency range from about 5 Hz to about 100 kHz with a presented impedance of at least 8 Ohms at 100 Hz.

Furthermore, application WO 2017002065 A1 relates to a magnetic stimulation device intended for the treatment of neurodegenerative diseases. This document discloses a method of exposure using a single coil capable of generating a peak magnetic flux density at the coil surface between 0.15 T and up to 7 T at repetition rates from 1 to 700 Hz. It further teaches the stimulation of neural structures for the treatment of neurodegenerative diseases with repetition rates exceeding 100 Hz, employing impulse durations in the range of 10 to 700 ps and requiring a magnetic flux density of at least 0.1 T for a purported therapeutic effect. However, the disclosed evidence fails to demonstrate any measurable or reproducible connection between the magnetic field exposure and a tangible therapeutic or cellular benefit.

In summary, the state of the art comprises various devices that generate magnetic fields for applications ranging from bone density improvement to muscle stimulation and tissue engineering. However, there remains an unmet need for therapeutic devices that are specifically adapted to treat neurodegenerative diseases, particularly rapidly deteriorating conditions such as motor neuron diseases such as ALS.

### OBJECT OF THE INVENTION / TECHNICAL PROBLEM

An objective of the present invention is to address a significant gap in the treatment of neurodegenerative diseases, particularly motor neuron diseases like ALS. Current therapeutic approaches do not effectively target the cellular impairments associated with these conditions. Thus, the invention aims to provide a device which is capable and especially configured for exposing a target with a spatial volume that includes motor neurons with a specially adapted transient magnetic field, enabling a targeted therapeutic intervention.

It is further an objection of the invention to provide an in vitro method to enhance neuronal activity, especially the speed of organelle transport within motor neurons. Such improvement may help mitigate symptoms of neuronal degeneration and support better motor function in subjects affected by, or at risk of, motor neuron diseases.

### SOLUTION

The problem is solved by a device as defined in claim 1, which comprises a magnetic field generation system configured to produce a transient magnetic field to a target. In parallel, the invention also provides a method for exposing a spatial volume containing motor neurons to a transient magnetic field, thereby increasing enhancing neuronal activity.

Additional advantageous embodiments and further developments are disclosed in the dependent claims and the detailed description with reference to the accompanying figures.

### GENERAL ADVANTAGES

Current treatments for severe neurodegenerative diseases such as ALS rely almost exclusively on the administration of drugs. The few drugs that have been approved can only provide minor relief and only marginally slow down the ultimately fatal course of the disease in most ALS patients. There is no known long-term treatment. In addition, the administration of drugs is associated with a number of side effects.

In contrast, the inventors were able to show in cell biological tests that the optimized fields of alternating (transient) magnetic field exposure are effective in this very area, restoring axonal organelle motility, mitochondrial functionality and regeneration.

The application of transient magnetic fields to the entire motor nervous system is a unique invention of the inventors, as no such system adapted to the specific conditions are known to date. Existing and established medical procedures and devices use static magnetic fields (e.g. in magnetic resonance imaging - MRI) or magnetic fields applied at higher frequencies (e.g. in transcranial magnetic stimulation - TMS), in some cases with significantly higher amplitudes. In particular, these procedures do not target the motor nervous system in the periphery (lower motor neurons).

The apparatus and method disclosed herein exhibit a number of general advantages. Firstly, it offers a novel, evidence-based approach to direct cell transport stimulation for diseases, especially ALS, for which there is currently no cure and few therapeutic options, all of which only marginally slow the disease progression. The apparatus provides magnetic field generation devices adapted to target the entire neuronal system, in particular the motor neuron system with the peripheral lower motor neurons and the upper motor neurons centered on the spine and head.

Through a comprehensive series of cell biological experiments on cultured human motor neurons from ALS patients, the inventors have identified a method that utilizes pulsed or continuously oscillating magnetic fields within a defined low-frequency range. This method has been shown to directly stimulate or reactivate inactive motor neurons with a high degree of efficiency. This method has been shown to facilitate the sustainable reactivation of axonal organelle transport, axonal growth velocity, and regenerative capacity, in addition to the DNA repair process within the cell nucleus, through specific exposure to magnetic fields. Moreover, it is an important achievement of the inventors to have clearly linked the beneficial impact of this method on the abovementioned cellular events to reproducible alterations in gene expression profiles through a genome-wide transcriptome analysis, thereby deciphering the cellular response upon the magnetic field stimulation on the underlying level of gene expression regulation.

Moreover, the therapeutic effect observed in motor neurons, particularly in terms of axonal organelle transport, persists for a duration exceeding a week following the final exposure, thereby providing a foundation for the development of efficient therapeutic exposure schemes for the treatment of patients.

The inventors' achievement in identifying spatial distributions and orientations of the field that interact with motor neurons in an especially efficient manner, and in developing an apparatus capable of providing this specially adapted pulsed or continuously oscillating magnetic field on a target volume, such as a cell, a part of or a whole organism, to enhance motor neuron activity, is clearly unprecedented.

### DETAILED DESCRIPTION

The invention pertains to an apparatus, in particular to a directional controlled magnetic treatment apparatus, alternatively preferably an apparatus arrangement, configured to expose a body comprising a target, preferably a body comprising a target, more preferably a body comprising multiple targets wherein between 30% and 100% of the targets are exposed, to a magnetic field, comprising:
- a support structure, whereby the support structure is preferably adapted to spatially arrange and support the magnetic field generation devices, the mounting device and the target,
- a mounting device configured to receive and/or support the target, preferably configured as a non-magnetic mounting device,
- at least one magnetic field generation device adapted to generate a transient magnetic field, preferably between 2 and 100 magnetic field generation devices,
- a control and supply circuit, adapted to supply power to and/or control at least one parameter of the transient magnetic field,

wherein the target is positioned at a target location, the target location being at a defined distance from the magnetic field generation device, wherein fixed distance preferably is between 0.01 cm and 1 m, more preferably between 0.1 cm and 50 cm, and
wherein the target is surrounded by a body of a spatial volume, the body having a specific magnetic permeability, wherein a specific magnetic permeability is between 1.0×10⁻⁶ H/m to 1.5×10⁻⁶ H/m, and wherein the target comprises or consists of neurons, preferably motor neurons with axons,
wherein the transient magnetic field is a pulsed and/or oscillating magnetic field with a repetition frequency and a flux density,
**wherein**
   - the flux density of the magnetic field is spatially adjustable, and
   - the flux density of the magnetic field at the target location is between 5 mT and 100 mT, preferably between 7 and 75 mT, and
   - the repetition frequency of the magnetic field is between 1 Hz and 1000 Hz, in particular between 1 and 200 Hz, preferably between 5 Hz and 100 Hz, more preferably between 10 Hz and 50 Hz, most preferably between 15 Hz and 40 Hz, and
   - the magnetic field is preferably substantially perpendicular to the target, more preferably the magnetic field is substantially perpendicular to the mean axonal plane and/or the target plane, wherein substantially perpendicular preferably means that the magnetic field is at an angle of 0° to 40° to the surface normal of the mean axonal plane and/or to the surface normal of the target plane, and
   - wherein exposure by the apparatus preferably leads to a measurable increase in neuronal cell activity (neuronal activity) comprised by the target or a target consisting of neurons, on a cellular level, as for example determined with live fluorescence microscopy image series.

Thus, in the spirit of the invention, the apparatus provides a system especially configured for providing a magnetic field which is delivered in an optimized manner both for in vitro applications (ex vivo), such as for neuronal cell cultures or tissue slices, and for treatment applications, in particular related to neuronal degenerative diseases, in a macroscopic target (in vivo), such as a human subject, which is accomplished by an increase in neuronal cell activity and/or DNA repair activity, as further elaborated herein. This may lead to positive therapeutic effects for a person whose body is exposed to the transient magnetic field of the device, for example a person suffering from a neurodegenerative disease whose symptoms related to a decrease in neuronal cell activity may be alleviated by the device and whose survival time could be prolonged.

A "target location" is a fixed location, wherein the target or the body comprising the target is positioned in or on the mounting device. The target location provides a defined distance from the magnetic field generation device, preferably between 0.01 cm and 1 m, more preferably between 0.1 cm and 50 cm, which allows for a planned and characterizable magnetic field exposure. Preferably, the mounting device provides a shape or positioning means to arrange the target or the body comprising the target in a defined way. In some embodiments, the apparatus comprises means to determine the target location automatically, via a sensor, and adjust the magnetic field provided by the magnetic field generation device(s) accordingly.

A "support structure", as defined in this document, is a component of the apparatus which provides structural support, positioning, actuation and fixing means adapted to spatially arrange and support the components of the apparatus. This includes, but is not limited to, the following: the magnetic field generation device(s); further magnetic field generation device(s); the control and supply circuit; connection means for the control and supply circuit; housing; visualization and diagnostic systems; user interfaces and the mounting device.

A support structure may also provide the means for a cooling system disposed or mounted on the magnetic field generation device(s). A cooling system is thermally coupled to the magnetic field generation device(s), preferably the cooling system comprises multiple conduits and/or is integrated into the mounting device. This increases the potential electrical power to be converted into the magnetic field.

In certain embodiments, the support structure incorporates movable components that enable the adjustment of both the magnetic field generation devices and the mounting device to match the dimensions of the target body. For human subjects, this support structure may be wearable or partially mobile, allowing the subject to move during extended exposure sessions lasting several hours.

In other preferred embodiments, the apparatus comprises a limited number of magnetic field generation devices, preferably between one and ten, while multiple such apparatuses may be arranged together to deliver transient magnetic fields to complex bodies with varied target orientations (e.g., different axonal axes or planes). This configuration can be powered by either a single unified control and supply circuit or by separate circuits, with the latter option offering a simpler power infrastructure compatible with standard power sources. An "apparatus arrangement" as disclosed herein comprises multiple apparatuses, preferably between 2 and 10, that share common unified and/or decentralized control and supply circuits. This configuration offers advantages such as simplified transport, more efficient power provision, and improved adaptability to various body forms, which is particularly beneficial when the target is a subject suffering from or at risk for neurodegenerative disease.

In some most preferred embodiments related to the transient magnetic field exposure of a subject, the apparatus or apparatus arrangement provide the means to target 30 to 100%, more preferably 50 to 100%, of all targets in the subjects body with an transient magnetic field in accordance with the current invention, in particular with a transient magnetic field that is a pulsed and/or oscillating magnetic field with a repetition frequency and a flux density which is spatially adjustable, and the flux density of the magnetic field at the target location is between 5 mT and 100 mT, preferably between 7 and 75 mT, and the repetition frequency of the magnetic field is between 1 Hz and 1000 Hz, in particular between 1 and 200 Hz, preferably between 5 Hz and 100 Hz, more preferably between 10 Hz and 50 Hz, most preferably between 15 Hz and 40 Hz, and the magnetic field is preferably substantially perpendicular to the target, more preferably the magnetic field is substantially perpendicular to the mean axonal plane and/or the target plane, wherein substantially perpendicular preferably means that the magnetic field is at an angle of 0° to 40° to the surface normal of the mean axonal plane and/or to the surface normal of the target plane. This allows the provision of a magnetic field which effectively may stimulate most of the neurons inside of the targets of the subject, such as lower and upper motor neurons.

Additionally in a wearable embodiment of the apparatus, the apparatus features mounting devices specifically designed to enclose the body comprising a target and provide an attachment interface for the support structure. This support structure includes movable components, and actuation means that facilitate movement during treatment, thereby enabling seamless integration into the subject's daily routine.

A "mounting device" configured to receive and/or support the target as defined herein. The mounting device comprises a surface, which refers to the outer interface layer of the mounting device directed towards the target. The mounting device is designed to arrange the spatial volume of the body surrounding the target so that the target is positioned within a pre-established, reproducible area. It is further designed to provide a small distance, preferably between 1 mm and 5 cm, between the magnetic field generation device(s) and the body comprising the target. Preferably, the mounting device and its surface are non-magnetic.

In some preferred embodiments, where the target has no direct contact with the device, the mounting device refers to the means which position, arrange or fix the magnetic field generating devices in alignment with the target. This may be the case when the support structure is attached to the ground or a otherwise provide the structural integrity for the apparatus and the magnetic field generation device(s) are placed in regard to the target and the body comprising it. Herein, the mounting device provides the means or orienting the target in relation to the magnetic field generation devices in order to allow precise tuning of the magnetic field orientation, especially in regard to the axonal axis or plane comprised by a target.

The term "non-magnetic" may be understood as including diamagnetic and paramagnetic properties, e.g., having a magnetic susceptibility in the range between -1 · 10⁻⁴ to +1 · 10⁴, alternatively in the range between -0.05 and +0.05. This has the technical advantage of not interfering with or shielding the transient magnetic field of the present invention.

In preferred embodiments, the magnetic field generation devices are integrated into or arranged directly adjacent to the material of the mounting device. This allows a short, defined distance to the target, as the magnetic field strength decreases to the power of two or three, depending on the distance.

In preferred embodiments, the mounting device is planar or substantially planar. Here, the maximum mean deviation from an ideal median plane is limited to no more than 20%, preferably no more than 10%, of the maximum lateral extension of the device. For example, if the maximum extension of the mounting device in the plane is 100 mm, the deviation should not exceed 20 mm, preferably not exceed 10 mm. Such a planar design provides an ergonomically optimized interface, particularly for human subjects, and facilitates positioning the magnetic field generation devices in a perpendicular or substantially perpendicular orientation relative to the mounting surface. Additionally, arrangements may include parallel configurations of further magnetic field generation devices (e.g., Helmholtz coils) to create complex magnetic field profiles with both orthogonal and parallel gradients relative to the target plane.

In alternative embodiments, the mounting device may feature a contoured (or "distorted") surface, such as those found in specially designed beds or chairs. These surfaces are ergonomically optimized to conform to the body's shape and still maintain a defined target location. In these cases, each magnetic field generation device is arranged in a perpendicular or substantially perpendicular orientation relative to the local surface contour. Therefore, this embodiment can be interpreted as a distorted planar mounting device adapted to the surface of the body of a target, especially a human body.

In the spirit of the invention, herein the term "target" refers to a physical object that is situated within a body which possesses a spatial volume as well as a specific magnetic permeability. The target comprises or consists of neurons, preferably motor neurons with axons. A target may be selected from the ex vivo or in vivo targets, preferably from the group consisting of cells, cell clusters, micro-organs, tissues, organs, parts of living organisms, or entire living organisms. For example, in certain embodiments the target may be an isolated neuronal cell culture, an engineered neural tissue, a brain slice, or a three-dimensional neural organoid. In the context of this invention, a target is defined by its distinct spatial dimensions and orientation. For neural cells, the target is characterized by a mean axonal axis and/or plane along which the majority of neurons are aligned. Since neural cells in living organisms (in vivo) exhibit unique distribution patterns, different clusters or regions, such as those found in a human body, can be regarded as separate targets, each with its own specific axonal orientation and dimensions.

As used herein, "specific magnetic permeability" refers to the intrinsic magnetic property of the materials that make up the target, which quantifies the degree to which the target allows the formation of an internal magnetic field when exposed to an external magnetic field. In preferred embodiments, this parameter is defined by a permeability coefficient, which may be expressed in Henrys per meter (H/m) or as a dimensionless relative permeability. In particular, the permeability coefficient in the context of the body comprising the target is in the range of 1.0×10⁻⁶ H/m to 1.5×10⁻⁶ H/m, which can be determined with a magnetometer, such as a superconducting quantum interference device (SQUID) magnetometer, by calculating the ratio of the measured induced magnetization to the applied magnetic field.

In some embodiments related to methods for enhancing neuronal activity in vitro, the target is an ex vivo-target, which are not considered as living, sentient targets, preferably selected from the group consisting of isolated cells, cell clusters, microorganisms, tissue sections, or isolated organs. This approach provides a model for optimizing parameters and arrangements for an apparatus, thereby ensuring that the most effective pulsed and/or oscillating magnetic field is applied to enhance neuronal activity. These embodiments especially related to "in vitro" methods, which are conducted in ex vivo-targets.

In alternative embodiments associated with an apparatus configured to expose a target to a magnetic field, the target may be selected from both living (i.e., in vivo) and ex vivo (i.e., in vitro) specimens. Living targets may include eukaryotic cells (such as primary neuronal cells or established neuronal cell lines), microorganisms, lower vertebrates or invertebrates used in research, small mammals (such as rodents), and humans.

In the context of this patent application, the term "subject" denotes one or more individuals or groups of individuals who suffer from, or are at risk of suffering from, a neurodegenerative disease affecting neurons, preferably motor neurons. For instance, in particularly preferred embodiments, a subject may be an individual suffering from amyotrophic lateral sclerosis (ALS) or a similar condition. Moreover, the subject may be selected from a gender- and/or age-restricted group, such as human adults between 18 and 120 years of age, more preferably between 30 and 100 years (typically onset times of ALS are associated with increasing age). A subject contains numerous targets within the body, as the nervous system, especially the motor neuron system comprising both upper and lower motor neurons, extends from the brainstem to peripheral regions. Consequently, delivering a magnetic field to one or, preferably, all of these targets poses a significant challenge. This challenge is effectively addressed by the apparatus, or an arrangement of apparatuses, as described in the current invention.

In some preferred embodiments of the invention, the target is in a body of a subject or a group of subjects suffering or at risk from suffering from a neurodegenerative disease, preferably a neurodegenerative disease selected from the list consisting of amyotrophic lateral sclerosis (ALS), primary lateral sclerosis (PLS), progressive muscular atrophy (PMA), spinal muscular atrophy (SMA), Alzheimer's, Parkinson's, Huntington's, Creutzfeldt-Jakob Disease (CJD) or diabetic polyneuropathy. These conditions are characterized by complex, not fully understood pathogeneses and currently lack universally effective treatments or cures. Consequently, employing an apparatus as described herein, either as a standalone therapy or in combination with existing treatments, may offer a novel therapeutic approach by addressing the underlying cellular dysfunction, potentially alleviating discomfort, improving functionality and prolonging survival time of an affected subject. This is directly related to the effect of the transient magnetic field generated (provided) by the device on neuronal cell activity and/or DNA repair activity of the target cells, in particular neurons, such as motor neurons, in the patient's body.

Herein, a "body" refers to a contiguous physical mass or material structure that encloses, surrounds, or supports the target. The body is characterized by a defined spatial volume and a specific magnetic permeability, which influences the local distribution and intensity of the applied magnetic field. In some embodiments, particularly for in vitro applications, the body may correspond to a support matrix, medium, or substrate in which cell cultures, tissue sections, or organoids are maintained. In alternative embodiments, especially in macroscopic treatment applications, the body refers to the physical structure of a living organism or a portion thereof (in vivo target), such as the human torso, limb, head, or whole body. The technical advantage of taking the body into account when configuring the apparatus is that its specific magnetic permeability can be taken into account in order to optimize the exposure of the target to the desired magnetic field parameters. A body in the spirit of the current invention may comprise more than one target.

In some preferred embodiments, where the apparatus is configured to expose a body containing multiple targets, it delivers a transient magnetic field to a defined proportion of these targets-between 10% and 100%, preferably 30% to 100%, and most preferably 50% to 100%. Here, "providing a transient magnetic field" refers to applying a pulsed or oscillating field with a specified repetition frequency, flux density, and orientation relative to the target plane, as disclosed herein. This may be determined by performing field measurements using a target dummy with measuring points and comparing the results with anatomical atlases and simulations of neural cell distributions in a magnetic field-exposed body.

Herein, the term "neuron" is employed to designate a specialized biological cell that transmits electrical signals, commonly referred to as action potentials, across a neural network within the nervous system. These cells are primarily located in the brain and spinal cord and are essential for receiving, processing, and conducting impulses. Although various types of neurons exist - such as sensory neurons and interneurons - the focus of this invention is on those neurons involved in motor functions.

In the context of the present invention, the term "neuron" encompasses both healthy and pathologically altered cells. "Healthy" neurons refer to cells obtained from individuals or cultured in vitro that display normal physical and genetic characteristics without any detectable abnormalities. In contrast, "damaged or mutated" neurons are those derived from subjects who exhibit genetic alterations or physical impairments, particularly associated with neurodegenerative conditions. This definition specifically includes cells affected by neuronal diseases, with an emphasis on motor neuron disorders such as amyotrophic lateral sclerosis (ALS). By adopting this broad definition, the invention is applicable to a wide range of diagnostic and therapeutic strategies targeting both normal and diseased neural cells. It is an exceptional achievement of the inventors to have found a transient magnetic field apparatus which is able to both stimulate and activate healthy and damaged cells, whereby the effects in damaged cells are more pronounced.

In the spirit of the invention, the term "motor neuron" refers to a specialized neuron whose cell body is located in the motor cortex, brainstem, or spinal cord. The axon of a motor neuron extends from the central nervous system to the peripheral target sites, thereby controlling effector organs, predominantly muscles, either directly or indirectly. Motor neurons are typically categorized into "upper motor neurons," which originate in the motor cortex and project to lower motor centers, and "lower motor neurons," whose cell bodies reside in the brainstem or spinal cord and whose axons extend to innervate skeletal muscles. Additional examples of motor neuron targets include neuromuscular junctions in the limbs, facial muscles, and respiratory muscles.

The spatial organization of motor neurons in relation to the muscles they innervate is of particular relevance in the context of this invention: In the context of the spinal cord, the cell bodies of lower motor neurons, also known as alpha motor neurons, are situated in the ventral horn, and their arrangement is characterized by somatotopy, also referred to as a "musculotopic" map. Medially located motor neuron pools innervate the axial muscles of the neck and trunk and the proximal limb muscles, whereas laterally located pools innervate the distal muscles of the limbs. A dorsoventral pattern is also evident: motor neurons that control flexor muscles are positioned more dorsally in the ventral horn, while those innervating extensor muscles lie more ventrally. Individual muscles are served by distinct clusters of motor neurons, called motor pools, which form longitudinal columns spanning one to several spinal segments. The medial motor neuron column traverses the majority of the spinal cord, providing innervation to the axial musculature. In contrast, the lateral columns are exclusive to the cervical and lumbosacral enlargements, corresponding to the limb muscles of the arms and legs.

The spatial arrangement of motor neurons can be mapped using diffusion tensor magnetic resonance imaging (DTI). This measurement method, which is a form of magnetic resonance imaging (MRI), involves the mapping of the diffusion of water molecules in tissue, thereby providing insight into the structure and orientation of white matter tracts. In white matter, water diffusion exhibits high anisotropy, meaning directional inequality, due to the restriction of water movement across the fibers by axonal membranes and myelin sheaths, while allowing for easier diffusion along the length of the fibers. DTI has been demonstrated to reveal the orientation of motor neurons. For instance, DTI measurements of the spinal cord demonstrate that white matter tracts are predominantly oriented along the longitudinal (rostrocaudal or craniocaudal) axis of the cord (Ellingson et al.; Diffusion Tensor MR Imaging of the Neurologically Intact Human Spinal Cord; American Journal of Neuroradiology Aug 2008, 29 (7) 1279-1284; DOI: 10.3174/ajnr.A1064).

Thus, in preferred embodiments of the present invention, the precise mapping of the spatial organization of motor neurons using DTI enables the design of an apparatus configured to expose the target to a spatially adjustable, pulsed and/or oscillating magnetic field, especially a magnetic field perpendicular to the axonal plane. The resulting technical benefit is an enhanced ability to selectively stimulate motor neurons, potentially increasing axonal transport and neuronal cell activity, which is especially advantageous for therapeutic interventions in neurodegenerative diseases such as amyotrophic lateral sclerosis (ALS).

The "axonal plane" in the spirit of the current invention is defined as the predominant orientation of motor neuron axons, particularly the axonal axis orientation, within a given spatially defined environment and/or anatomical region. The axonal plane is defined as the mean orientation of the axonal axes - that is, the average longitudinal direction of the majority of axons within the spatially defined environment.

In some embodiments, wherein the targets are in vitro targets, such as cell cultures, motor neurons often exhibit a preferred or tailored direction for axonal outgrowth, and the axonal plane is defined as the mean orientation of these axonal projections. This defined plane facilitates the application of a magnetic field that is substantially perpendicular to the axonal extensions, thereby enhancing the effectiveness of the field in promoting axonal transport and neuronal activity. The technical benefit is that optimizing the field orientation relative to this axonal plane can improve the stimulation of the neurons and potentially accelerate intracellular processes.

In embodiments relating to a macroscopic target, such as a whole organism or parts thereof, the "axonal plane" is defined as the predominant orientation of motor neuron axons within a given anatomical region and/or spatially defined environment. Such anatomical region and/or spatially defined environment are known to the person skilled in the art from anatomical studies or can be determined using specialized imaging techniques such as DTI.

In preferred embodiments pertaining to humans and upper motor neurons, these axons run primarily along the longitudinal (rostrocaudal) axis of the body, for example in the spinal cord or brainstem. Alternatively, for lower motor neurons, the axonal plane may be defined by the nerve tracts that extend from the spinal cord to the target muscles, with the common mean orientation of the axons determined as a numerical approximation of the average axonal direction within that region. The technical benefit is that such precise mapping allows for magnetic field generation devices to be arranged so as to produce a field substantially perpendicular to the axonal plane, thereby optimizing therapeutic stimulation.

A "target plane" in this context, particularly in the case of a whole human target, is defined as the horizontal plane formed by the frontal and longitudinal axes - or anatomically the "coronal plane" - passing through the spine and extending through the shoulders. This target plane serves as the reference for positioning and orienting the magnetic field, ensuring that the field is applied in a manner that is substantially perpendicular, with a deviation of 0 to 40° from the surface normal of the axonal plane. The technical advantage of this precise orientation is to maximize the influence of the magnetic field on the motor neurons, thereby potentially enhancing axonal transport and improving the overall therapeutic outcome in neurodegenerative diseases.

In the context of this invention, the term "substantially perpendicular" refers to the relative orientation between the applied magnetic field and the defined axonal plane and/or the target plane. "Substantially perpendicular" means that the magnetic field is applied at an angular deviation of between 0° and 40°, more preferably between 0° and 30°, most preferably between 0° and 25°, from the surface normal of the axonal plane. In other words, a 0° deviation indicates that the magnetic field is exactly orthogonal to the axonal plane, while a deviation up to 40°, preferably 30°, most preferably 25° is deemed acceptable to maintain the technical benefit of optimal stimulation of motor neurons in the complex setting of a biological system. The technical benefit of applying the magnetic field in a substantially perpendicular orientation is that it maximizes the interaction between the field and the motor neuron axons, thereby enhancing axonal transport and neuronal activity, which is especially advantageous for therapeutic interventions in neurodegenerative diseases.

In some preferred embodiments, the exposition of the target by the magnetic field increases the axonal organelle mobility by at least 20%, preferably between 20% and 500%, more preferably between 30 % and 300%, in particular in damaged or mutated motor neuron cells, as determined by video fluorescence microscopy measurements. Axonal organelles are cellular vesicular components transported along neuronal axons, especially in motor neurons, and include lysosomes, endosomes, and mitochondria. Their transport occurs in the anterograde direction via kinesin motors and in the retrograde direction via cytoplasmic dynein and dynactin complexes. These transport processes can be quantified in in vitro setups comprising a distal axon compartment connected to a proximal somatic compartment via microchannels, thereby enabling precise measurement of axonal orientation, plane, and organelle transport speeds.

In some preferred embodiments, the exposition of the target by the magnetic field increases the axonal organelle mobility by at least 20%, preferably between 20% and 100%, of healthy cells, preferably healthy motor neuron cells. This effect has been demonstrated on a number of different experiments and may be used in activity stimulation of in-vitro (ex vivo) samples as well as in living organisms (in vivo).

In some preferred embodiments, the transient magnetic field generated by the present invention produces quantifiable cellular effects, especially related to DNA damage response of cells, such as neurons. In these embodiments of the current invention the DNA repair activity of damaged cell nuclei in targets comprising neurons, preferably motor neurons, preferably measured in total turnover of the FUS repair protein at the DNA damage site (in the formal unit "fold change of FUS protein amount at the DNA damage site over background multiplied by seconds), is increased by at least 20%, preferably by 20% to 300%, compared to targets, such as cells, in particular neurons, not exposed to the field, as determined by fluorescence microscopy. For example, when cell nuclei are deliberately damaged using a microscopic laser scalpel, preceding exposure to the transient magnetic field accelerates DNA repair, also referred to as DNA repair activity or repair turnover, in cells, in particular mutated cells, such as the ALS FUS cells of the examples disclosed herein. This enhanced DNA repair activity promotes cellular regeneration and may contribute to slowing the progression of neurodegenerative diseases.

In some further preferred embodiments, the transient magnetic field generated by the present invention produces quantifiable cellular effects, wherein the cellular effect is an increase a neuronal cell activity, in particular axonal organelle transport activity, selected from the list consisting of mean velocity, track displacement, moving fraction, ratio of anterograde to retrograde tracks, by at least 20%, preferably between 20% and 500%, preferably between 30% and 400%, as determined by video fluorescence microscopy measurements. This allows neural stimulation in healthy and mutated or damaged cells on the cellular level, which may therefore enable the treatment of neuronal degenerative diseases.

A "magnetic field generation device" in the spirit of the current invention is a device or an arrangement of centrally controlled device(s), preferably between 2 and 100 magnetic field generation devices, adapted to generate a transient magnetic field, in particular a pulsed and/or oscillating magnetic field with a repetition frequency and a flux density as described herein.

In a preferred embodiment of the apparatus, the magnetic field generation device(s) are provided in an arrangement of 2 to 100 magnetic field generation device(s), preferably between 20 and 80 magnetic field generation devices and 2 to 4 further magnetic field generation devices. The arrangement of magnetic field generation devices also referred to as magnetic field generation array, arranged below the surface mount and perpendicular to the target, in particular to the coronal plane of the target, with which magnetic pulses of duration around 0.02 - 0.1 s (corresponding to a frequency between 10 - 50 Hz) may be generated.

In preferred embodiments, the magnetic field generation array comprises a scalable quantity of 2 to 100 magnetic field generation device(s) mounted in the support structure, arranged directly on/below the mounting device. These devices are positioned so that they generate magnetic fields substantially perpendicular to the plane of a reclining or sitting body comprising the target, in particular a human body.

In some preferred embodiments of the magnetic field generation devices, designed as electromagnetic coils, the magnetic field generation devices have an inductance (also referred to as coil inductance) of less than 100 mH (e.g., less than 50 mH, e.g., less than 10 mH) and/or varies from a common reference inductance by less than 5 Millihenry (mH) and/or less than 5% of the common reference inductance, wherein, preferably, the reference inductance is between 0.5 and 50 mH (e.g., between 1 and 5 mH). This facilitates providing a transient magnetic field at a repetition frequency between 1 Hz and 1000 Hz, in particular between 1 and 200 Hz, preferably between 5 Hz and 100 Hz, more preferably between 10 Hz and 50 Hz, most preferably between 15 Hz and 40 Hz.

In some preferred embodiments, the magnetic field generation devices arranged at the mounting device are provided at a specific density of between 3 and 100 devices per square meter (m²) of the mounting device surface, preferably between 5 and 50 devices per square meter (m²). This increases the resolution for controlling the magnetic field.

In some preferred embodiments the apparatus comprises an arrangement of 2 to 100 magnetic field generation devices positioned substantially perpendicular in relation to the mean axonal plane and/or the target plane, wherein substantially perpendicular preferably means that the magnetic field is at an angle of 0 to 40° to the surface normal of the mean axonal plane and/or to the surface normal of the target plane.

In preferred embodiments, the magnetic field generation device(s) is/are selected from the group comprising electromagnetic helix or spiral coils (solenoid coils), air coils, solid-core coils, Helmholtz coils, racetrack coils, or any combination thereof. This selection enables efficient and adaptable delivery of the magnetic field to targeted regions within a body. A skilled practitioner can employ established calculation and adjustment methods to achieve the required field orientation and modulation as disclosed herein.

In embodiments addressing complex bodies with targets in varying orientations, such as a human body, the devices may be strategically arranged around the target. For example, providing a Helmholtz or racetrack coil configuration with a mounting device with places a certain body part, such as an arm or a leg inside of such a coil, can yield a more homogeneous magnetic field in that body comprising the target.

In additional preferred embodiments, the apparatus incorporates one or more further magnetic field generation devices, preferably between 2 and 10 further magnetic field generation devices, also referred to as "additional coils." These coils are selected from Helmholtz, racetrack, or air coils with diameters sized to encompass at least one body part of the target, and are configured preferably freely positionable, which means that the support structure and the mounting device provide means to adjust the position of these further magnetic field generation devices in regard to the body comprising the target. The further magnetic field generation devices are arranged to envelop either the entire body or specific body parts, and they are oriented to produce magnetic fields parallel to the target plane (coronal plane) or the longitudinal (craniocaudal) axis. In embodiments targeting specific regions within a subject's body, a magnetic field device may be positioned around an arm or leg, such as a racetrack coil or two Helmholtz coils aligned with the subject's longitudinal axis.

Furthermore, some embodiments may incorporate specially designed coils with optimized geometries, such as cylindrical coils or racetrack coils. These are configured to generate higher magnetic field amplitudes specifically in targeted regions, for example, the arms, legs, or head area. In particular, these coils can be placed around the parts of the body that comprise the target, providing a very defined and homogeneous field.

An "control and supply circuit" is a circuit system adapted to supply power to and/or control at least one parameter of the transient magnetic field. The control and supply circuit according to the current invention comprises
- a capacitance circuit for providing a capacitance,
- a power generation circuit configured to provide electrical power; and
- an operating circuit comprising one or more output terminals for outputting the electrical power in accordance with a plurality of operating modes, of which

wherein the operating modes comprises a pulsed mode of operation alternating between a first phase and a second phase,
wherein the capacitance circuit is coupled to the power generation circuit to charge the capacitance by the electrical power in the first phase, and wherein the capacitance circuit is coupled to the one or more output connectors to discharge the capacitance via the one or more output connectors in the second phase;
   - wherein in at least one continuous operation mode, coupling the power generation circuit to the one or more output connectors to supply the electrical power to the one or more output connectors, and
   - wherein control and supply circuit provides the electric power to generate the transient magnetic field according to the current disclosure, especially a pulsed and/or oscillating magnetic field with a repetition frequency between 1 Hz and 1000 Hz, in particular between 1 and 200 Hz, preferably between 5 Hz and 100 Hz, more preferably between 10 Hz and 50 Hz, most preferably between 15 Hz and 40 Hz, and a flux density between 5 mT and 100 mT, preferably between 7.5 and 75 mT.

The magnetic field generation device(s) may be operated in a power-optimized manner using the control and supply circuit as detailed herein. Here, the control and supply circuit may provide multiple operating modes, in particular to generate high pulsed, preferably in the range of about 100 to 1000 A, and/or continuously oscillating, preferably between 100 and 500 A electric currents. In the pulse operation mode (e.g., generating a pulsed current), the supply and control circuit provides a peak electrical output power between 100 kW and 1000 kW, preferably between 100 and 500 kW.

However, conventional magnetic field generation devices utilizing conventional control and supply circuits, such as those employed in medical technology, for example in magnetic resonance imaging (MRI) or transcranial magnetic stimulation (TMS), are not designed or configured to generate pulsed and/or oscillating magnetic fields with a repetition frequency between 1 Hz and 1000 Hz, in particular between 1 and 200 Hz, preferably between 5 Hz and 100 Hz, more preferably between 10 Hz and 50 Hz, most preferably between 15 Hz and 40 Hz, and a flux density between 5 mT and 100 mT, preferably between 7.5 and 75 mT. MRI magnets and their power supplies are electromagnetically too inert, due to high inductances, to realize relevant magnetic field changes, especially in the appropriate frequency interval. Such mostly superconducting systems, i.e. relying on liquid helium cooling, are optimized solely for the provision of temporally constant magnetic fields. In contrast, TMS magnet systems are of low-inductance design and their high-power power supplies are optimized for the generation of correspondingly short magnetic field pulses (e.g., less than 1 ms), i.e. corresponding frequencies of more than 1 kHz.

In the state of the art, different magnet sources are used in various applications, which can be categorized in two distinct forms:
A vector magnet source is a device or system designed to generate a controlled magnetic field with a predetermined direction and magnitude. Such devices can produce either uniform or non-uniform magnetic fields, making them essential for applications in scientific research, materials testing, and medical imaging (e.g., magnetic resonance imaging). By precisely controlling the overall field orientation and strength, these sources enable detailed manipulation of magnetic environments.

In contrast, a field gradient source is engineered to create a magnetic field whose strength varies spatially. This spatial variation - or gradient - is crucial in applications requiring precise localization, such as magnetic sensing, targeted magnetic particle manipulation, and certain imaging techniques. A magnetic field gradient creates forces on magnetized microscopic (e.g. magnetic moments of ions or free radicals in proteins) and macroscopic objects (e.g. pieces of permanent magnets, of soft magnetic materials, of anisotropic diamagnetic organic materials).

Within the present invention, a "transient magnetic field" - alternatively referred to as an "alternating magnetic field" - is a pulsed and/or oscillating magnetic field characterized by a specific repetition frequency and flux density. This field is generated by one or more magnetic field generation devices and is distinguished by a spatially adjustable flux density that can be tailored to the target location and orientation within a body. Such adjustability is critical for interfacing with both simple and complex biological systems, including those with intricate or variable neural cell arrangements.

In some embodiments, the transient magnetic field generated by the present invention comprises a static component-referred to as the ground field strength-that serves as the baseline around which the pulsed and/or oscillating field alternates. In one preferred configuration, the static field is set to 0 mT, causing the transient field to oscillate symmetrically (for example, alternating from +5 mT to -5 mT when the amplitude is 10 mT).

Alternatively, the static field strength may be adjusted within a range of 0 mT to 50 mT to superimpose a constant baseline field with the transient component, thereby enabling additional biological effects. Furthermore, in certain embodiments, the sum of the maximum flux density and the static field is maintained at or below 100 mT to reduce power consumption while still achieving the desired therapeutic impact.

The flux density of the magnetic field, also referred to as the field amplitude, at the target location is between 5 mT and 100 mT, preferably between 7.5 and 75 mT. Preferably, the flux density of the magnetic field at the target location is in a range of two of the endpoints selected from the list consisting of 5 mT, 6 mT, 7 mT, 8 mT, 9 mT, 10 mT, 11 mT, 12 mT, 13 mT, 14 mT, 15 mT, 20 mT, 25 mT, 30 mT, 35 mT, 40 mT, 45 mT, 50 mT, 60 mt, 70 mT, 80 mT, 90 mT, and 100 mT. This range has been demonstrated to enhance axonal organelle transport, promote motor neuron regeneration, and sustain regenerative effects for 7 days or more in both healthy and ALS-affected cells. Flux density is typically measured directly using devices such as gaussmeters, pickup coils, or fluxgate magnetometers. In embodiments where the waveform includes harmonic components, indirect measurement methods such as time-domain analysis via oscilloscopes, frequency counters integrated with magnetic field sensors, or spectrum analyzers are employed.

The repetition frequency of the magnetic field is between 1 Hz and 1000 Hz, in particular between 1 and 200 Hz, preferably between 5 Hz and 100 Hz, more preferably between 10 Hz and 50 Hz, most preferably between 15 Hz and 40 Hz. Preferably, the repetition frequency is in a range of two of the endpoints selected from the list consisting of 1 Hz, 2 Hz, 3 Hz, 4 Hz, 5 Hz, 6 Hz, 7 Hz, 8 Hz, 9 Hz, 10 Hz, 11 Hz, 12 Hz, 13 Hz, 14 Hz, 15 Hz, 16 Hz, 17 Hz, 18 Hz, 19 Hz, 20 Hz, 21 Hz, 22 Hz, 23 Hz, 24 Hz, 25 Hz, 26 Hz, 27 Hz, 28 Hz, 29 Hz, 30 Hz, 31 Hz, 32 Hz, 33 Hz, 34 Hz, 35 Hz, 36 Hz, 37 Hz, 38 Hz, 39 Hz, 40 Hz, 41 Hz, 42 Hz, 43 Hz, 44 Hz, 45 Hz, 46 Hz, 47 Hz, 48 Hz, 49 Hz, and 50 Hz. This range has been shown to be effective in improving axonal organelle transport, motor neuron regeneration and sustained regeneration for 7 days or more in healthy and ALS cells.

In some particularly preferred embodiments, the repetition frequency has been associated with an unprecedented, surprisingly strong neuronal response at the cellular level as displayed in the examples disclosed herein, that has not been previously described, resulting in a particularly strong effect when transient magnetic fields are applied, especially in the 10 to 50 Hz range.

In some preferred embodiments the magnetic field has a modulation wave function selected from the list consisting of sinusoidal wave, square wave, bi- or multiphasic square wave. This modulation flexibility allows the apparatus to generate magnetic fields that are either continuously oscillating or applied in pulses. In some most preferred embodiments, the transient magnetic field has a modulation wave which is represented of a sinusoidal wave or a modified sinusoidal wave.

A "pulsed magnetic field" as contemplated by the present invention is delivered in discrete bursts or pulses. These pulses can be represented by nonsinusoidal and even square waveforms with a defined duty cycle. Preferably, the duration of each pulse is maintained within the range of approximately 0.02 to 0.1 seconds.

An "oscillating magnetic field" in this context is one that periodically reverses its polarity in a continuous manner. The archetypal example is a sinusoidal waveform. Alternatively, the oscillating field may be implemented using bi- or multiphasic square waves. In preferred embodiments, the oscillation frequency (repetition rate) is maintained within the range of 10 to 50 Hz.

The current invention also pertains to a **method** for enhancing neuronal activity in vitro using an apparatus, especially enhancing axonal organelle transport in healthy and unhealthy cells, the method comprising the following steps:
a) Providing a target
b) comprising or consisting of neurons, in particular motor neurons with axons,
c) Placing the target on a mounting device, then
d) Exposing the target to a pulsed and/or oscillating magnetic field during a fixed time interval, preferably between 10 µs and 12 h, wherein the magnetic field has a flux density and a repetition frequency, wherein the flux density is between 5 mT and 100 mT and the repetition frequency is between 1 Hz and 1000 Hz, in particular between 1 and 200 Hz, preferably between 5 Hz and 100 Hz, more preferably between 10 Hz and 50 Hz, most preferably between 15 Hz and 40 Hz , then preferably rest the target for a fixed time interval, preferably between 10 µs and 1 h,
e) Repeat step c several times to reach a total procedure time of 10 min to 12 hours, wherein
   - the target comprises, preferably is, an in vitro neuron, preferably an in vitro motor neuron,
   - the target is comprised in a volume, preferably between 1 µm³ and 0.5 m³,
   - the volume has a spatial elevation above the surface, preferably between 10 µm and 50 cm,
   - the volume has a specific permeability, preferably between 1.0×10⁻⁶ H/m to 1.5×10⁻⁶ H/m.

It is a great achievement of the inventors to have found a method of applying a pulsed and/or oscillating magnetic field with a repetition frequency and flux density capable of restoring axonal organelle mobility, mitochondrial functionality, DNA damage response and regeneration in motor neurons with a clear correlation to significant, defined alterations in the underlying gene expression profiles, as revealed by genome-wide transcriptomics. In some embodiments, the method employs continuous transient magnetic field exposure, wherein step (c) is repeated without interruption, achieving a truly continuous application. In alternative embodiments, the transient magnetic field is delivered as a finite sequence of pulses or oscillations following a defined pattern, which is then interrupted by a rest period or a switch to another pattern.

Preferred embodiments may combine various pulsed and/or oscillating magnetic field waveforms-such as sinusoidal, square, and bi- or multiphasic square waves-by executing step (c) in different sequences with optional rest or transition intervals between patterns.

In a most preferred embodiment, the method utilizes a continuous sinusoidal transient magnetic field, which has been shown to activate neural effects in vitro, such as increased axonal organelle mobility.

In some preferred embodiments of the method for increasing neuronal activity in vitro according in which the target comprises or consists of motor neurons with axons, wherein the magnetic field increases a neuronal cell activity, in particular axonal organelle transport activity, selected from the list consisting of mean velocity, track displacement, moving fraction, ratio of anterograde to retrograde tracks, by at least 20%, preferably between 20% and 500%, preferably between 30% and 400%, as determined by video fluorescence microscopy measurements. This allows neural stimulation in healthy and mutated or damaged cells on the cellular level.

Furthermore, the invention pertains to an exposure plan for exposing a target, preferably an in vitro target comprising neurons to a magnetic field with an apparatus according to the current disclosure, with a method as described herein, wherein the method is applied in fixed time intervals, wherein the fixed time interval is between 1 day and 2 weeks, wherein the gap between the time intervals may be referred to as rest time. This allows to utilize the recovery effect of the magnetic field exposure, which may be 8 days or more. By periodically exposing the target to the magnetic field, the neuronal activity enhancing effect can be maintained with minimal exposure repetitions.

For the purposes of this disclosure, the terms "connected," "attached," and "coupled" refer to either direct or indirect physical or electrical linkages, including ohmic or conductive connections. In circuit diagrams, such connections are indicated by solid lines.

"Coupled" or "coupling" denotes any form of interaction or linkage, whether mechanical, hydrostatic, thermal, or electrical, between elements, enabling the exchange of interactions such as power, force, or signals, regardless of whether the connection is direct or indirect.

Components are considered electrically conductive if their conductivity exceeds approximately 1 S/m, preferably exceeds 10³ S/m, more preferably 10⁸ S/m, whereas they are deemed electrically insulating if their conductivity is below approximately 10⁻⁶ S/m, preferably below 10⁻⁸ S/m, more preferably below <10⁻¹⁰, most preferably below 10⁻¹⁴S/m.

A circuit component (such as a capacitor, resistor, or coil) may represent an electrical property (capacitance, resistance, inductance) provided by a single device or by multiple devices connected in series, parallel, or combinations thereof, with the aggregated property denoted by the respective component.

"Processes" include any sequence of operations or methods performed by hardware components, by computer-readable instructions executed on a processor, or by a combination of both, where the functions may be implemented in software, firmware, or specialized circuitry.

A "processor" is any entity that handles data or signals, including analog circuit, digital circuit, mixed-signal circuit, logic circuit, processor, microprocessor, Central Processing Unit (CPU), Graphics Processing Unit (GPU), Digital Signal Processor (DSP), Field Programmable Gate Array (FPGA), integrated circuit, Application Specific Integrated Circuit (ASIC), or any combination thereof, that performs designated functions according to programmed instructions.

A "control device" (or controller) is any logic-implementing entity-whether special-purpose circuitry or a processor executing software-that directs system operation, potentially incorporating hard-wired or programmable logic and memory to store instructions, rules, or algorithms.

"Memory" refers to any non-transitory computer-readable medium for storing data or information, including both volatile and nonvolatile types (such as RAM, ROM, flash memory, solid-state drives, hard disk drives, optical media, registers, or buffers), where a single memory component may encompass multiple memory types or be subdivided into equivalent components.

"Controlling" means intentionally influencing the state of a system or process, termed the actual state, to align with a desired target state by adjusting one or more operating parameters (manipulated variables) via actuators or regulators. Closed-loop control refers to a system in which the actual state is continuously monitored and compared with the target state, with deviations corrected in real time by adjusting operating parameters through feedback, thereby minimizing the error between the current and desired states.

An "actuator" is the interface between a control device and a controlled entity that adjusts a specific parameter based on the control device's output. It converts control signals, particularly electrical, into an action (mechanical, electrical, etc.) that modifies the controlled entity. Actuators can take the form of motors, solenoids, hydraulic or pneumatic cylinders, semiconductor switches, or other mechanisms, including circuit elements (e.g., switches, diodes, thyristors, relays) that reconfigure a circuit to alter conductive paths or control signals.

The term "channel" denotes a pathway through which interactions, such as electrical, logical, pneumatic, hydraulic, or electromechanical, are transmitted. This transmission may occur via physical means, such as electromagnetic signals over wires or wireless links, or through logical software-level connections that facilitate data exchange. In electrical circuits, a channel typically refers to a conductive path that carries currents, voltages, or power, while in data communication it represents the underlying medium supporting the logical exchange of information.

Further advantages, features and possible applications of the present invention are also apparent from the following description of embodiments and the drawings. All the features described and/or illustrated form the object of the present invention, either individually or in any combination, even independently of their summary in the claims or their references.

The features mentioned in the claims and in the description can each be essential to the invention individually or in any combination.

### EXAMPLES

The present invention is explained in more detail with reference to the following figures and embodiments, without limiting the invention to these. In particular, features shown in the individual figures and described for the respective example are not limited to the respective individual example.

Examples of embodiments are shown to explain the basic principle of the device according to the invention. It should be noted that the ratio, the dimensions, the extent of deformation or the amount of displacement of the components according to the invention do not correspond to reality for the sake of illustration.

The singular includes the plural, unless the context clearly indicates otherwise. Certain terms and derivations thereof are used in the following description for convenience only and are not intended to be limiting. For example, words such as "up", "above", "upward", "down", "below", "downward", "left" and "right" refer to directions in the drawings to which reference is made, unless otherwise indicated. Similarly, words such as "inward" and "outward" refer to directions directed toward or away from the geometric center of a device or area or specific parts thereof. References in the singular include the plural and vice versa, unless otherwise indicated.

The features, characteristics and advantages of the present invention described above and the manner in which they are achieved will be more clearly understood in the context of the following description of the embodiments. Where the term "may" is used in this application, it refers to both the technical possibility and the actual technical implementation.

In the following, the invention will be described in detail by referring to exemplary embodiments that are accompanied by figures, in which:
- **Fig. 1A**: shows a neuron (2.3), here a motor neuron (2.4) having an axon (2.5).
- **Fig. 1B**: illustrates axonal organelle transport along a microtubule (2.5.1) in axons (2.5).
- **Fig. 2A**: illustrates a first view of a target (2.0).
- **Fig. 2B**: illustrates a second view of a target (2.0).
- **Fig. 3A**: illustrates an apparatus (1.0) in the spirit of the current invention, as viewed from below.
- **Fig. 3B**: illustrates an apparatus (1.0) in the spirit of the current invention, as viewed from the side.
- **Fig. 3C**: illustrates an apparatus (1.0) in the spirit of the current invention, as viewed from the top.
- **Fig. 3D**: illustrates an apparatus (1.0) in the spirit of the current invention, as viewed from the front.
- **Fig. 3E**: illustrates an example of an apparatus (1.0) in the spirit of the current invention, as viewed from the front.
- **Fig. 3F**: illustrates a further example of an apparatus (1.0) in the spirit of the current invention, from an oblique angle.
- **Fig. 4A/4B**: shows the mean speed in µm/s (y-axis) of lysosomes/mitochondria (left/right) plotted over repetition frequency (x-axis) after exposure to a magnetic field of the current invention.
- **Fig. 4C/4D**: shows the track displacement in µm (y-axis) of lysosomes/mitochondria (left/right) plotted over repetition frequency (x-axis) after exposure to a magnetic field of the current invention.
- **Fig. 4E/4F**: shows the moving fraction in % (y-axis) of lysosomes/mitochondria (left/right) plotted over repetition frequency (x-axis) after exposure to a magnetic field of the current invention.
- **Fig. 4G/4H**: shows the ratio of anterograde over retrograde tracks (y-axis) of lysosomes/mitochondria (left/right) plotted over repetition frequency (x-axis) after exposure to a magnetic field of the current invention.
- **Fig. 5A/5B**: shows the mean speed in µm/s (y-axis) of lysosomes/mitochondria (left/right) plotted over repetition frequency (x-axis) after exposure to a magnetic field of the current invention, comparing the impact of perpendicular against parallel orientation of the field lines relative to the axonal plane.
- **Fig. 5C/5D**: shows the track displacement in µm (y-axis) of lysosomes/mitochondria (left/right) plotted over repetition frequency (x-axis) after exposure to a magnetic field of the current invention, comparing the impact of perpendicular against parallel orientation of the field lines relative to the axonal plane.
- **Fig. 5E/5F**: shows the moving fraction in % (y-axis) of lysosomes/mitochondria (left/right) plotted over repetition frequency (x-axis) after exposure to a magnetic field of the current invention, comparing the impact of perpendicular against parallel orientation of the field lines relative to the axonal plane.
- **Fig. 5G/5H**: shows the ratio of anterograde over retrograde tracks (y-axis) of lysosomes/mitochondria (left/right) plotted over repetition frequency (x-axis) after exposure to a magnetic field of the current invention, comparing the impact of perpendicular against parallel orientation of the field lines relative to the axonal plane.
- **Fig. 6A/6B**: shows the mean speed in µm/s (y-axis) of lysosomes/mitochondria (left/right) plotted over time in days (x-axis) after exposure to a magnetic field of the current invention.
- **Fig. 6C/6D**: shows the track displacement in µm (y-axis) of lysosomes/mitochondria (left/right) plotted over time in days (x-axis) after exposure to a magnetic field of the current invention.
- **Fig. 6E/6F**: shows the moving fraction in % (y-axis) of lysosomes/mitochondria (left/right) plotted over time in days (x-axis) after exposure to a magnetic field of the current invention.
- **Fig. 6G/6H**: shows the ratio of anterograde over retrograde tracks (y-axis) of lysosomes/mitochondria (left/right) plotted over time in days (x-axis) after exposure to a magnetic field of the current invention.
- **Fig. 7A/7B**: shows the mean speed in µm/s (y-axis) of lysosomes/mitochondria (left/right) plotted over flux density in mT (x-axis) after exposure to a magnetic field of the current invention.
- **Fig. 7C/7D**: shows the track displacement in µm (y-axis) of lysosomes/mitochondria (left/right) plotted over flux density in mT (x-axis) after exposure to a magnetic field of the current invention.
- **Fig. 7E/7F**: shows the moving fraction in % (y-axis) of lysosomes/mitochondria (left/right) plotted over flux density in mT (x-axis) after exposure to a magnetic field of the current invention.
- **Fig. 7G/7H**: shows the ratio of anterograde over retrograde tracks (y-axis) of lysosomes/mitochondria (left/right) plotted over flux density in mT (x-axis) after exposure to a magnetic field of the current invention.
- **Fig. 8**: shows a principal component analysis (PCA) plot of genome-wide transcriptomic expression profiles of healthy (Ctrl) and ALS cells (ALS) after exposure to a magnetic field of the current invention.
- **Fig. 9**: shows a cell culture (6.0) as a target (2.0).
- **Fig. 10A**: shows a first embodiment of a supply and control circuit (5.0).
- **Fig. 10B**: shows a second embodiment of a supply and control circuit (5.0).
- **Fig. 11**: shows DNA damage response (DNA repair activity) in nuclei of healthy (Ctrl) and ALS cells (ALS) after exposure to a magnetic field of the current invention by means of total turnover of the FUS repair protein at the DNA damage site in fold changes x sec (y-axis) as a function of the repetition frequency of the transient magnetic field (Hz)

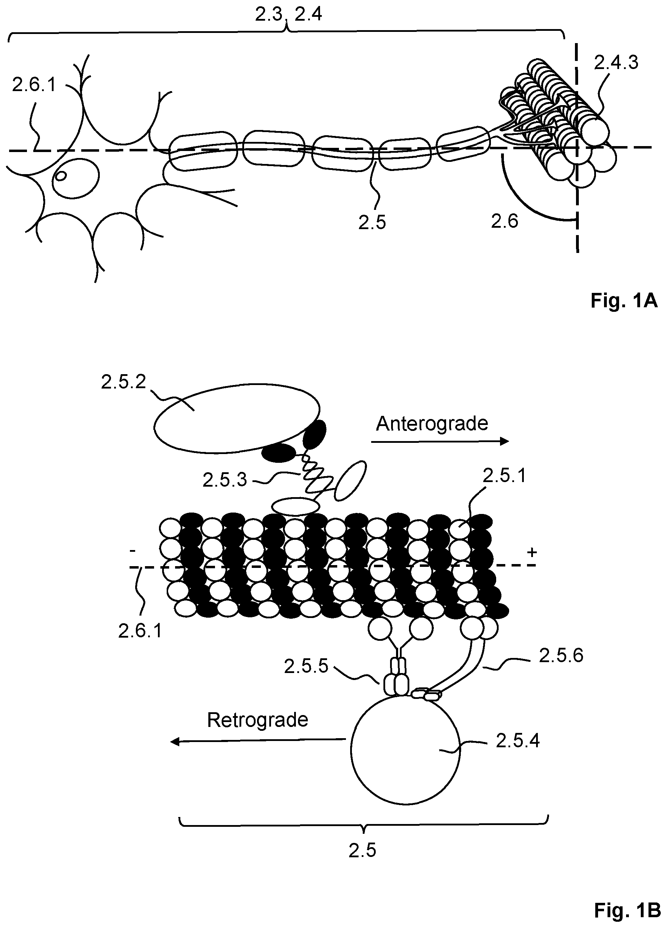

Figure 1A shows a neuron (2.3), here a motor neuron (2.4) connected to muscle fibers (2.4.3), with an axon (2.5) and an axonal plane (2.6), which is the plane opened by the axis of the muscle fibers (2.4.3) with the axonal axis (2.6.1), in this example. Figure 1B illustrates the axonal transport of organelles, the upper half shows the anterograde transport of mitochondria (2.5.2) by kinesin (2.5.3) along the axis of the microtubule (2.5.1). The lower half shows the retrograde transport of a lysosome (2.5.4) by a cytoplasmic dynein (2.5.5) and dynactin (2.5.6). The microtubule (2.5.1) has a potential gradient along the axonal axis (2.6.1).

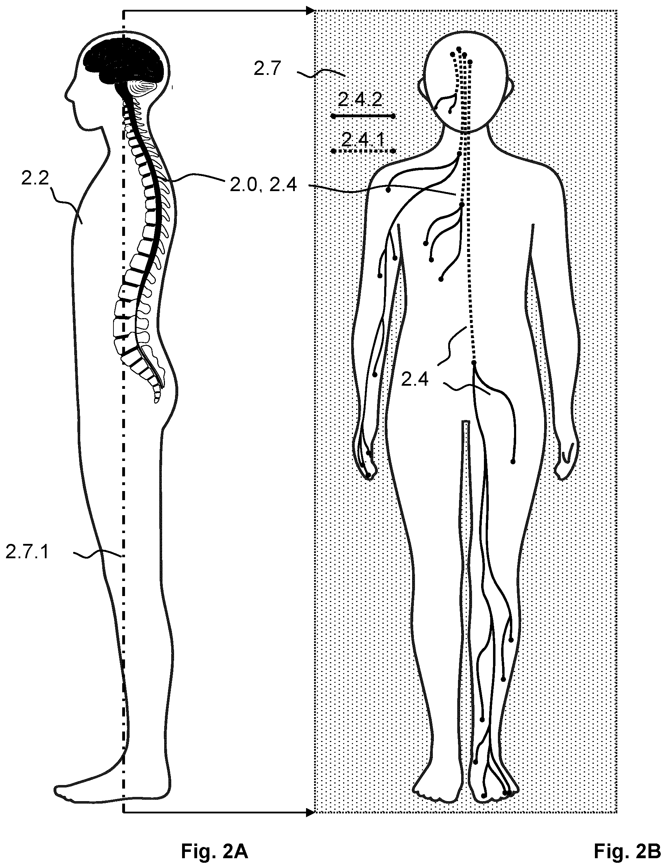

Figures 2A and 2B illustrate exemplary embodiments of a target (2.0) comprising motor neurons (2.4), here lower (2.4.2) and upper (2.4.1) motor neurons, comprised by a body (2.2) of a human. In general, the upper motor neurons (2.4.1) run along the longitudinal (craniocaudal) axis of a human (2.7.1), while the lower motor neurons (2.4.2) extend from the spinal region to the peripheral and central muscle fibers. The target body also has a target (coronal) plane (2.7).

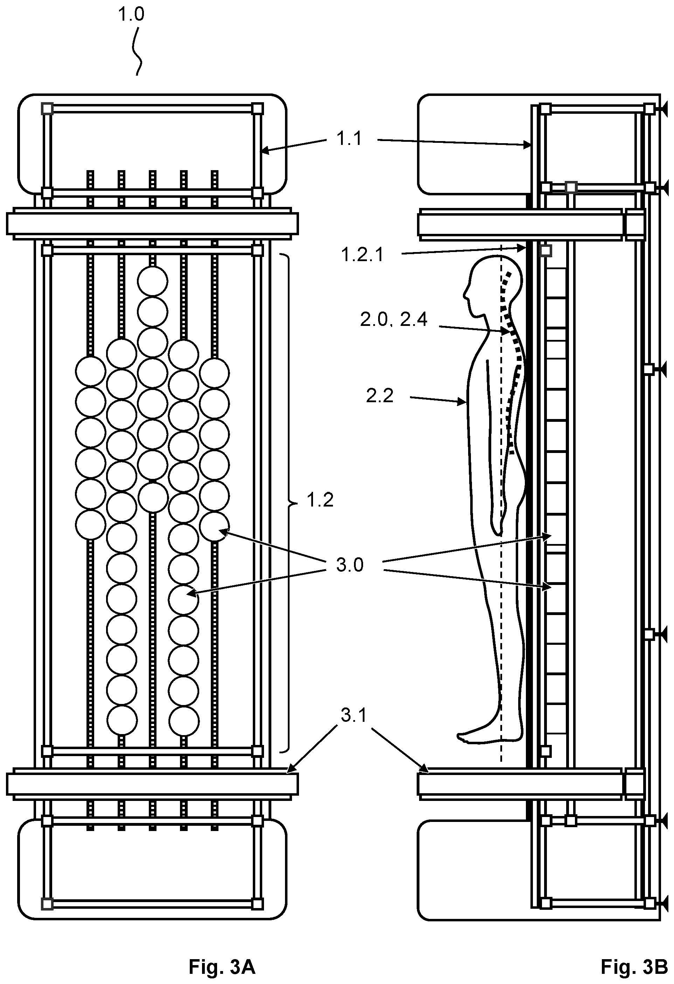

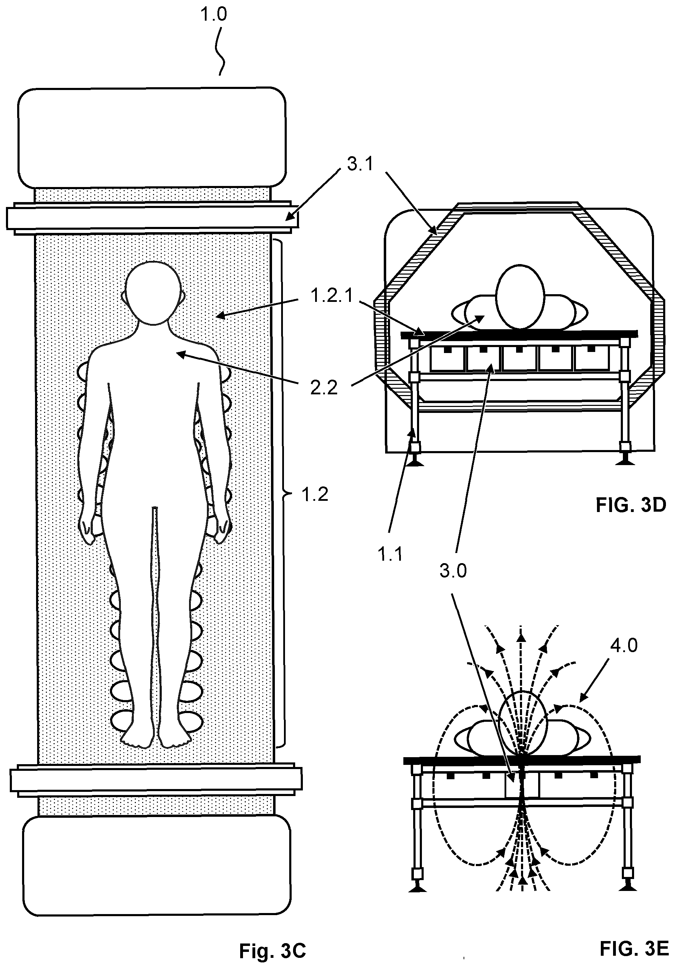

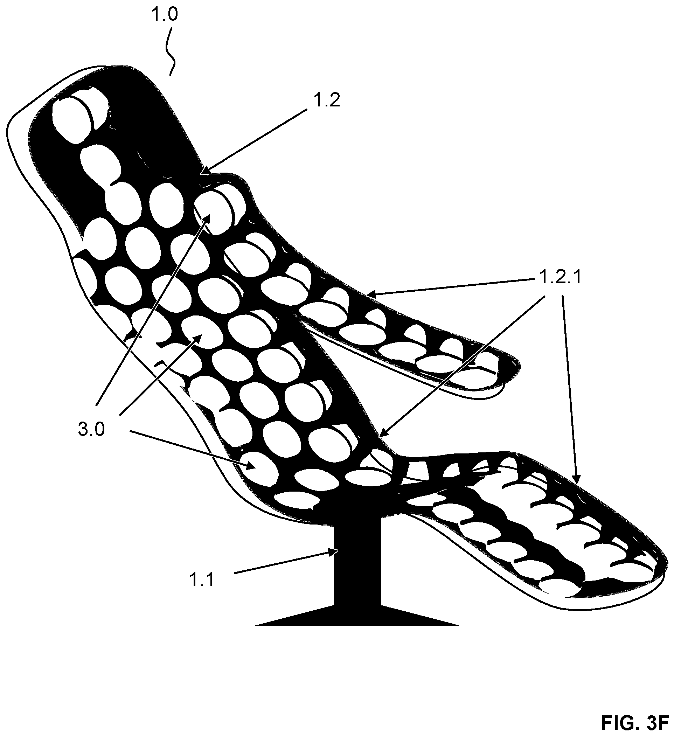

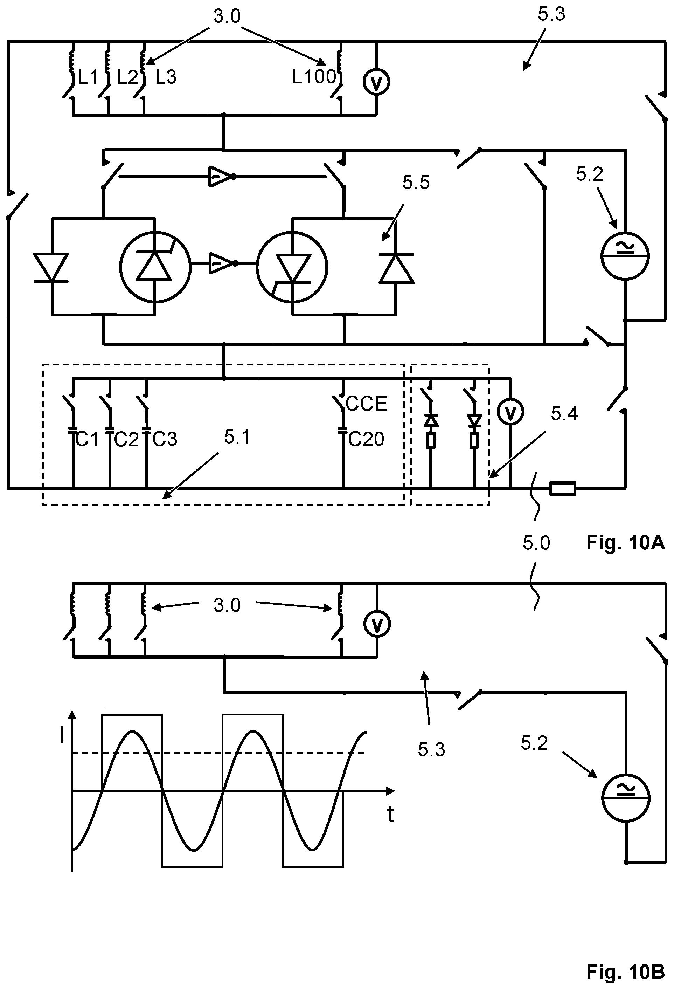

Figure 3 relates to the apparatus (1.0) of the present invention and illustrates exemplary embodiments of the apparatus (1.0). Figures 3A through 3F relate to embodiments of the apparatus in which the mounting device (1.2) has a substantially planar surface. Fig. 3A shows a view from below of the apparatus (1.0), Fig 3B the same apparatus from the side. This embodiment of the apparatus comprises a support structure (1.1), here as a profile system, supporting the mounting device (1.2), magnetic field generation devices (3.0) and further magnetic field generation devices (3.1) and being provided with a housing, here limiting the access of the target and users to the control and supply circuit (not shown here, illustrated exemplary as 5.0 in Fig. 10A/B) in order to reduce the risk of tampering or injury. In this example, the magnetic field generation devices (3.0) are electromagnetic coils adapted to generate a transient magnetic field (4.0). The magnetic field generators (3.0) are arranged in a coil array consisting of 46 magnetic field generators (3.0) arranged in 5 rows in such a way as to apply a magnetic field to the entire target or individual parts. This corresponds to a magnetic field generation device density of about 21 devices per square meter (m²) of the surface (1.2.1). In addition to the magnetic field generation devices (3.0), this example provides two further magnetic field generation devices (3.1) arranged parallel to the target plane (2.7) or the longitudinal axis (2.7.1) (see Figs. 2A/2B for reference). The two further magnetic field generation devices (3.1) are adapted to generate a magnetic field parallel to the plane, which allows to modulate the magnetic field (4.0) and to provide another exposure vector and are designed as Helmholtz coils. This embodiment includes an actuator coupled to the support structure and to the further magnetic field generation device(s) (3.1) and configured to change the position of the further magnetic field generation devices (3.1) relative to the surface (1.2.1) of the mounting device (1.2). The mounting device, herein made of a non-magnetic material, is configured to receive and/or support the target (2.0) comprising neural cells, herein the lower and upper motor neurons (2.4) in a human. The target (2.0) is surrounded by a body (2.2), specifically a human body comprising soft tissues, bones, with a spatial volume of approximately 0.07 m³.

In Fig. 3C, an example of the apparatus of Figs. 3A and 3B is shown, wherein the body (2.2) is positioned on the surface (1.2.1) of the mounting device (1.2), wherein in this example markings indicate the positions of the magnetic field generation devices (3.0) located directly below the mounting device (1.2), which allows for positioning of a subject's body (2.2) on the apparatus. Figure 3D shows a front view of the apparatus (1.0) of Figures 3A, 3B and 3C, illustrating an exemplary configuration of the further magnetic field generation device(s) (3.1).

Figure 3E shows a front view of an embodiment of the apparatus (1.0) with a single magnetic field generation device (3.0) and an illustrated magnetic field (4.0).

Figure 3F shows an embodiment of the apparatus (1.0) with the mounting device (1.2) having a contoured (or "distorted") surface (1.2.1), here designed as a chair. This surface (1.2.1) is ergonomically optimized to conform to the shape of the body while maintaining a defined target position. Here, each of the magnetic field generation devices (3.0) is arranged in a perpendicular or substantially perpendicular orientation relative to the local surface (1.2.1). The support structure (1.1) is integrated with the mounting device (1.2) to allow the field generation device(s) (3.0) to be positioned substantially perpendicular to the surface (1.2.1).

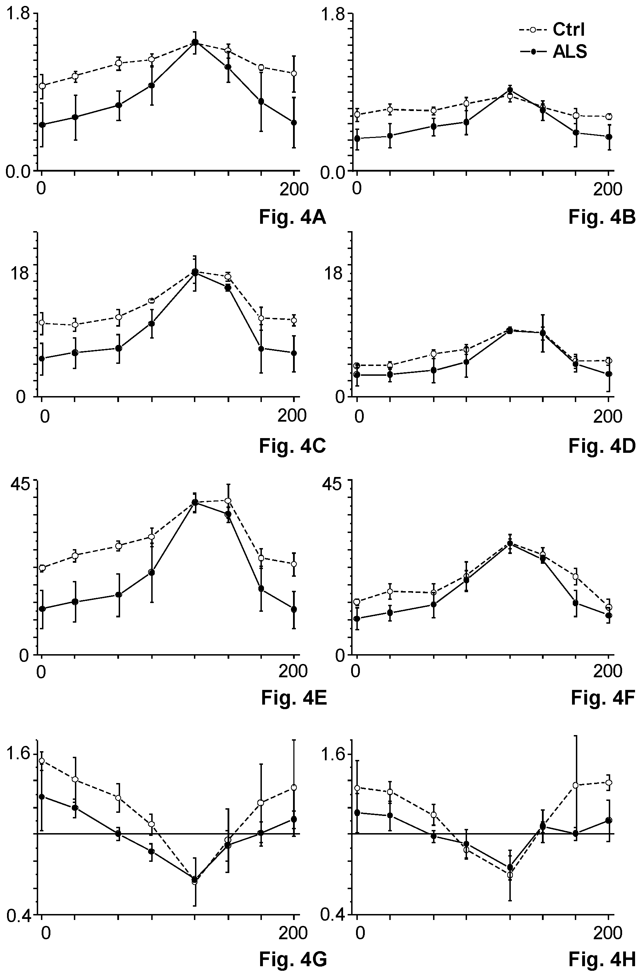

Figure 4 relates to an in vitro (ex vivo) cell culture application of the method according to the present invention and shows the influence of the repetition frequency, herein an alternate current (AC) repetition frequency, of the transient magnetic field exposure on the speed of axonal organelle transport (motility). In this example, FUS-GFP WT (Ctrl) motor neurons (MNs) were compared against isogenic mutant FUS-GFP P525L MNs (ALS), a cell culture of human ALS cells. All transient magnetic field exposures (MFE) were performed at 10 **mT** flux density and the indicated repetition frequency (x-axis, in Hz) and compared with unexposed cells (i.e., at 0 Hz). Individual organelles, here lysosomes (left column, Fig. 4A, 4C, 4E and 4G) and mitochondria (right column, Fig. 4B, 4D, 4F and 4H), in the entire pool of examined fluorescence microscopy image series (movies) were analysed with respect to four key tracking parameters (y-axis): mean speed in µm/s (Fig. 4A and 4B); track displacement in µm (Fig. 4C and 4D), moving fraction in % (Fig. 4E and 4F), and antero-/retrograde motility ratio (Fig. 4G and 4H) as a function of repetition frequency (x-axis) at a perpendicular orientation of the axonal plane to the magnetic field. Exposure according to the method disclosed herein was performed three times for 7 h every 5 days after cell maturation for 30 days (i.e., on day 30, 35, and 40) and finally once for 3.5 h on day 45. On day 48, live fluorescent video microscopy was performed to assess the axonal organelle motility. Data points are the mean of the total organelle population analysed, resulting in sample sizes typically in the range of tens of thousands across all pooled fluorescence microscopy image series (movies) per condition, i.e. for a given frequency and cell line, with error bars representing the standard deviation. This revealed a particularly effective range for all tracking parameters between 10 and 50 Hz, leading to an inversion of the net motion bias from anterograde to retrograde (Figs. 4G and 4H). This demonstrates the optimal repetition frequency range for neuron stimulation, quantified by the four parameters.

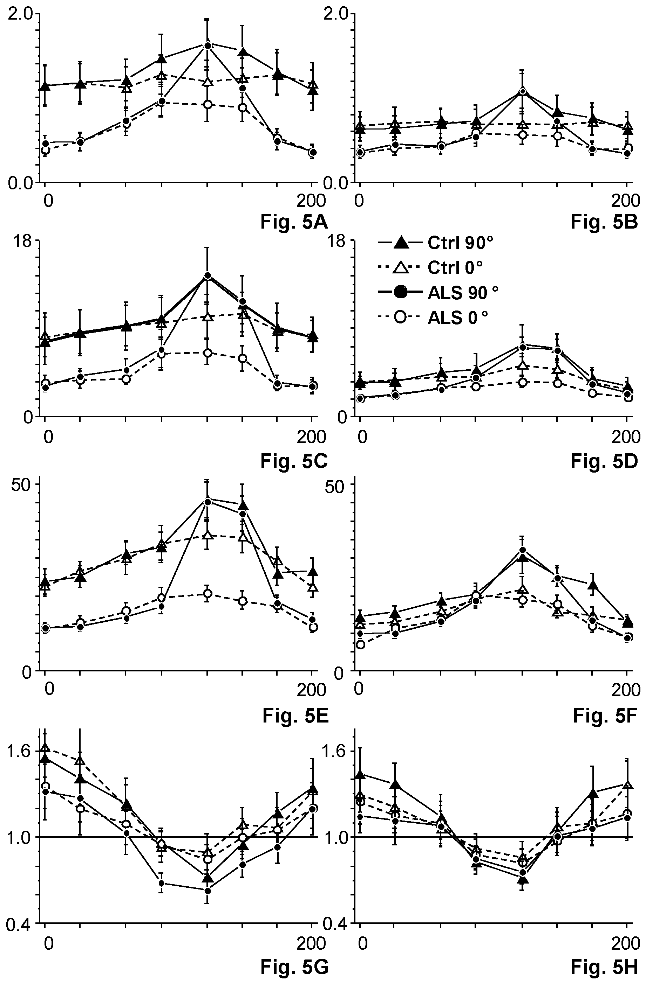

Figure 5 relates to an in vitro (ex vivo) cell culture application of the method according to the present invention and shows the influence of magnetic field orientation on the speed of axonal organelle transport (motility). In this example, FUS-GFP WT MNs (Ctrl) were compared against isogenic mutant FUS-GFP P525L MNs (ALS), a cell culture of human ALS cells. All exposures were performed at 10 **mT** flux density and the indicated repetition frequency (x-axis, in Hz) and compared to unexposed cells (i.e., at 0 Hz). Individual organelles, here lysosomes (left column, Fig. 5A, 5C, 5E and 5G) and mitochondria (right column, Fig. 5B, 5D, 5F and 5H), in the entire pool of examined fluorescence microscopy image series (movies) were analysed with respect to four key tracking parameters (y-axis): mean speed in µm/s (Fig. 5A and 5B); track displacement in µm (Fig. 5C and 5D), moving fraction in % (Fig. 5E and 5F), and antero-/retrograde motility ratio (Fig. 5G and 5H) as a function of repetition frequency similar to Fig. 4 (x-axis), but either in perpendicular (90°, solid curves) or parallel (0°, dashed curves) orientation of the axonal plane relative to the magnetic field. Exposure was performed three times for 7 h every 5 days after cell maturation for 30 days (i.e., on day 30, 35, and 40) and finally once for 3.5 h on day 45. On day 48, live fluorescent video microscopy was performed to assess the axonal organelle motility. Data points are averages of the total organelle population analysed, resulting in sample sizes typically in the range of tens of thousands across all pooled fluorescence microscopy image series (movies) per condition, i.e. for a given AC repetition frequency and cell line, with error bars representing the standard deviation. This revealed an particularly effective for the repetition frequency in perpendicular orientation (90°) for all tracking parameters in the preferred range between 10 and 50 Hz, corresponding to an inversion of the net motion bias from anterograde to retrograde (Figs. 5G and 5H), whereas transient magnetic field exposure (MFE) was much less efficient in parallel orientation (0°).

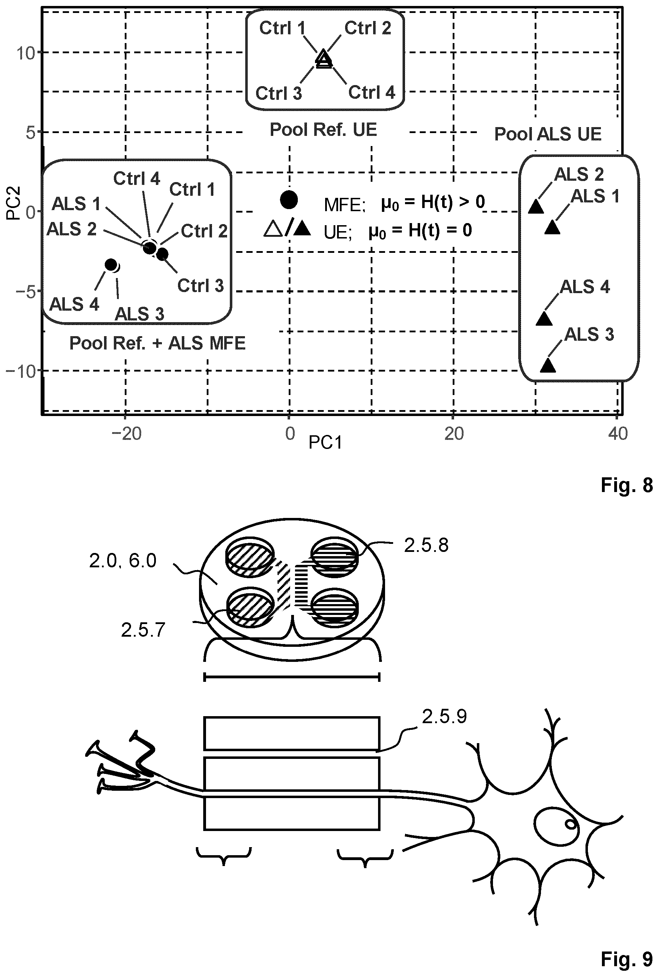

In detail: Figures 5A/5B show the mean speed in µm/s (y-axis) of either lysosomes (left) or mitochondria (right) plotted against repetition frequency (x-axis) in the series at perpendicular (90°, straight curves) versus parallel (0°, dashed curves) orientation. Figures 5C/5D show the track displacement in µm (y-axis) of either lysosomes (left) or mitochondria (right) plotted over repetition frequency (x-axis) in the series at perpendicular (90°, straight curves) versus parallel (0°, dashed curves) orientation. Figures 5E/5F show the moving fraction (y-axis), i.e., the percentage of the total organelle population defined as the mobile fraction showing a track displacement above a threshold of 1.2 µm as opposed to the immobile fraction below this threshold, of either lysosomes (left) or mitochondria (right) plotted over the repetition frequency (x-axis) in the series in perpendicular (90°, straight curves) versus parallel (0°, dashed curves) orientation. Figures 5G/5H show the ratio of anterograde to retrograde tracks. Due to the architecture (see Fig. 9) of the target (2.0), here the cell culture (6.0) microgroove (2.5.9), organelles can only move in one dimension within the axonal bundles, either anterogradely, i.e. towards the distal axonal tips in the distal axon compartment (2.5.7), or retrogradely, i.e. towards the proximal somadendritic site (2.5.8). Thus, each track within the mobile organelle fraction can be classified as either anterograde or retrograde. If an organelle changed direction during the movie acquisition, the overall net direction was used for classification.

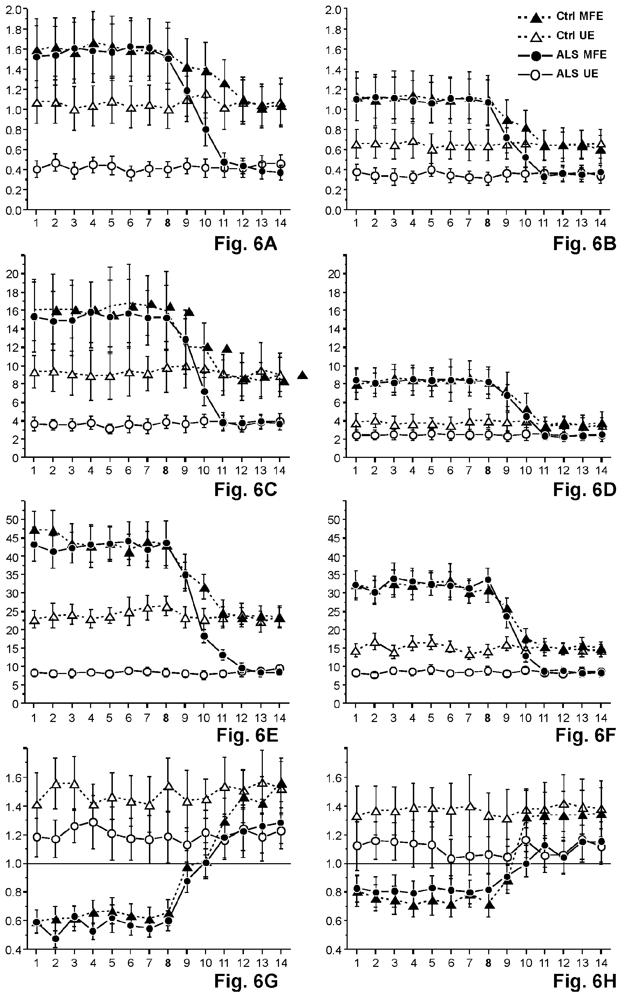

Figure 6 demonstrates the persistent effect of the transient magnetic field exposure (MFE) according to the present invention on the isogenic pair of FUS-GFP WT (Ctrl) and mutant FUS-GFP P525L MNs (ALS) compared to their respective unexposed (UE) counterparts beyond the last of the 4 transient magnetic field exposures (MFE) by quantitative tracking analysis of organelle motility. These measurements were performed analogously to Figure 4 (at an optimized AC repetition frequency in the preferred range between 10 and 50 Hz at 10 mT and perpendicular orientation), and the 4 tracking parameters (y-axis) were determined with fluorescence microscopy image series (movies): Mean velocity in µm/s (Figs. 6A and 6B); track displacement in µm (Figs. 6C and 6D), moving fraction in % (Figs. 6E and 6F), and antero-/retrograde motility ratio (Figs. 6G and 6H) as a function of chase time after final MFE in days (x-axis). Data points are the mean value of the entire organelle population analyzed, resulting in sample sizes typically in the range of tens of thousands across all pooled fluorescence microscopy image series (movies) per condition, i.e., for a given chase time and cell line, with error bars representing the standard deviation. Measurements were performed over a time series of 1-14 days (chase) after the last magnetic field exposure to determine the duration of the effect of magnetic field exposure (MFE). This example demonstrates the beneficial, stimulatory effect on the motility of both types of organelles (lysosomes and mitochondria) that was fully sustained beyond the final magnetic field exposure (MFE) session for at least 8 days in all tracking parameters (Fig. 6A to 6F; compare filled versus unfilled forms). Moreover, note that the stimulation of organelle motility in ALS neurons (Fig. 6A to 6F; compare filled versus unfilled circles) was even above the level of natural (UE) healthy donor neurons (Ctrl) (unfilled triangles) and that Ctrl neurons responded to magnetic field exposure in a similar way, i.e. they showed the same stimulated motility level (compare filled versus unfilled triangles). In the case of the antero-retrograde track ratio (Figure 6G/6H), the sustained stimulatory effect over 8 days corresponded to an inversion of the net movement bias from antero- to retrograde.

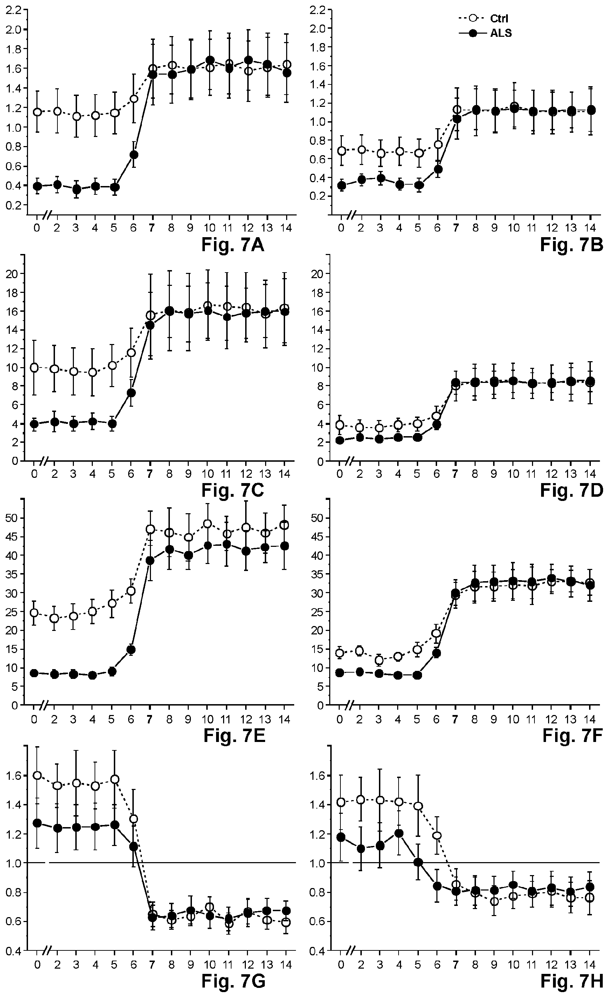

Figure 7 shows that the flux density of the magnetic field at a preferred AC repetition frequency range between 10 and 50 Hz and perpendicular orientation must be above a critical threshold of 5 mT, preferably at least 7 mT, to cause a significant stimulation effect on axonal organelle motility (lysosomes and mitochondria). These measurements were otherwise carried out in exactly the same way as in the preceding Fig. 4, but at different field strengths, at the same repetition frequency and perpendicular field orientation in relation to the axonal plane; and the 4 key tracking parameters (y-axis) were again deduced from fluorescence microscopy image series (movies): mean velocity in µm/s (Figs. 7A and 7B); track displacement in µm (Figs. 7C and 7D), moving fraction in % (Figs. 7E and 7F), and antero-/retrograde motility ratio (Figs. 7G and 7H) as a function of flux density in mT (x-axis). Data points are the mean values of the entire organelle population analyzed, resulting in sample sizes typically in the range of tens of thousands across all pooled fluorescence microscopy image series (movies) per condition, i.e., for a given flux density and cell line, with error bars representing the standard deviation. These measurements show a full stimulatory effect on the motility of both types of organelles (lysosomes and mitochondria) at 7 mT and higher in both lines (Ctrl & ALS) in all tracking parameters (Figs. 7A to 7F), whereas below 7 mT organelle motility remained indistinguishable from untreated cells, i.e., motility did not gradually increase with field strength, but suddenly switched at the critical threshold of 7 mT from an unstimulated low level to a stimulated higher level, even beyond untreated Ctrl cells. In the case of the antero-retrograde track ratio (Figs. 7G and 7H), the switch from low to high motility level corresponded to an inversion of the net movement bias from antero- to retrograde at 7 mT and higher field strengths.

Figure 8 presents a principal component analysis (PCA) plot summarizing the impact of transient magnetic field exposure (MFE) on genome-wide transcriptomic expression profiles (of top 500 most diverse genes) on spinal motoneurons in vitro. The culture, maturation and MFE of the neurons was exactly performed as for Fig. 4 onwards at a preferred repetition frequency in the range between 10 and 50 Hz, 10 mT and perpendicular orientation. Essentially, on day 48 after the final MFE at day 45, total RNA was extracted, purified and processed for next generation Illumina deep RNA sequencing. The analysis compares neurons from a pool of 4 healthy control probands (Ctrl, i.e. wild type) against a pool of 4 familiar ALS patients with mutations in the FUS gene (ALS), including the isogenic pair of FUS-GFP WT MNs (contained in Ctrl pool) and mutant FUS-GFP P525L (contained in ALS pool). The PCA plot reduces the complexity of multi-dimensional profiles, i.e., here of the of genome-wide transcriptomic expression profiles, down to 2 dimensions (PC1, y-axis, and PC2, x-axis), thereby clustering similar gene expression profiles closely together to reveal differences due to distinct genotypes and common cellular responses due to the MFE. PC1, accounting for 83% of the total variance, is plotted on the x-axis, while PC2, capturing 7% of the variance, is on the y-axis. The ALS group includes the following cell lines: ALS 1: FUS B1; ALS 2: FUS KG2; ALS 3: FUS 495x; and ALS 4: FUS-GFP P525L. The control group comprises: Ctrl 1: T12.9; Ctrl 2: Ctrl AKC5; Ctrl 3: Ctrl 30.1; and Ctrl 4: FUS-GFP WT. In the plot, triangles represent unexposed cells (UE), and circles indicate cells that were exposed to the magnetic field (MFE). Three distinct clusters are identifiable: one containing exclusively unexposed (UE) healthy control samples, one comprising only unexposed (UE) ALS patient samples, and a third group that includes both healthy and ALS samples that were exposed (MFE) to the transient magnetic field.

Figure 9 shows an example of a target (2.0), herein designed as a cell culture (6.0) with microgrooves (2.5.9), organelles can only move in one dimension within the axonal bundles, either anterogradely, i.e. towards the distal axonal tips in the distal axon compartment (2.5.7), or retrogradely, i.e. towards the proximal somatic side (2.5.8). Thus, each track within the mobile organelle fraction can be classified as either anterograde or retrograde. This allows to evaluate and quantify the exposure parameters in a simplified model of a biological system.

Figure 10A illustrates an embodiment of a schematic circuit diagram based on an exemplary implementation of the control and supply circuit (5.0). As illustrated, the capacitance circuit (5.1) may include a plurality (e.g., an amount C, e.g., 10 or more, e.g., 20 or more) of capacitors, the capacitance of which is denoted as C1 to CC (which here is C20 as an example); the capacitance circuit (5.1) may optionally include, for each of the plurality of capacitors, a circuit configuration element (CCE) (also referred to as capacity configuration elements) coupled in series with the capacitor. This allows the capacitance of the capacitance circuit (5.1) coupled to the operating circuit (5.3) to be manipulated, for example, by controlling the capacity configuration elements CCE. For example, the capacitance circuit (5.1) may comprise a plurality of subcircuits coupled in parallel, each of which comprises a series connection of a capacity configuration element CCE and a capacitor. The control and supply circuit (5.0) includes a plurality (e.g., an amount K, e.g., 10 or more, e.g., 20 or more) of coils, the inductance of which is denoted as L1 to LK (which here is L100 as an example). The power generation circuit (5.2) includes an AC/DC power supply configured to provide power of an AC type (e.g., an alternating current) and to provide power of a DC type (e.g., a direct current), e.g., in accordance with the determined operating mode. The operating circuit (5.3) includes multiple (e.g., a set of K or more) output connectors and, for each output connector, a circuit configuration element coupled in a series with the output connector. This allows manipulation of which output connector is supplied with electrical power, e.g., by controlling the output configuration elements. The operating circuit (5.3) may optionally include one or more additional circuit configuration elements (also referred to as operating mode configuration elements), the functionality of which is detailed with respect to the multiple (e.g., seven or more) operating modes. The optional pulse enable circuit (5.5) may couple (e.g., in series) the capacitance circuit (5.1) and the output connectors. The optional pulse damping circuit (5.4) is coupled in parallel with the capacitance circuit (5.1).

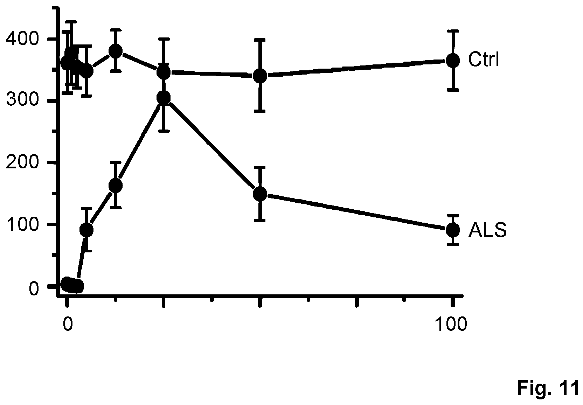

Figure 11 illustrates an exemplary effect related to DNA repair activity, in particular of the DNA repair activity of the transient magnetic field generated by an apparatus according to the present invention. The graph plots DNA damage response in spinal motor neurons in vitro by means of total turnover of the FUS repair protein at the DNA damage site in fold change over background x sec (y-axis) as a function of the AC repetition frequency of the transient magnetic field (Hz). The culture, maturation and MFE of the neurons was exactly performed as for Fig. 4 onwards at a flux density of 10 mT and perpendicular orientation. In this analysis, FUS-GFP WT MNs are compared against the isogenic FUS-GFP P525L ALS mutant at all indicated repetition frequencies (in Hz, x-axis) and versus unexposed cells (i.e., at 0 Hz). Nuclear DNA was precisely severed using a microscopic laser scalpel, and the subsequent DNA repair activity, here as repair turnover was quantified by video fluorescence microscopy.

### Design examples

With reference to the following figures and examples of embodiments, the present invention will be explained in more detail without limiting the invention thereto.

The following tables list some preferred shape-memory polymers comprised by the core fiber, Sample-ID-Nos.: 1 to 20 list some preferred, but not limiting, examples of shape-memory polymers of the invention, each of which is another embodiment of the present invention.

### Example 1:

This exemplary embodiment (see e.g. Figs. 3A to 3D) of the apparatus according to the present invention is an apparatus (1.0) configured to expose a target (2.0) to a magnetic field (4.0). The apparatus (1.0) is configured to expose a target (2.0) to a magnetic field (4.0), the apparatus (1.0) exemplarily comprising a supporting structure (1.1), herein a profile system, supporting the mounting device (1.2), magnetic field generating means (3.0), and provided with a housing, herein limiting the access of the target and users to the control and supply circuit (5.0) to reduce the risk of tampering or injury. The mounting device, herein made of a non-magnetic material, is configured to receive and/or support the target (2.0) comprising neural cells, herein the lower and upper motor neurons (2.4) in a human. The target (2.0) is surrounded by a body (2.2), specifically a human body comprising soft tissues, bones with a spatial volume of approximately 0.07 m³. In this example, the target (2.0) is positioned at a target location (2.1), the target location (2.1) being optionally marked with markings on the surface (1.2.1) of the mounting device (1.2) indicating the positions of the magnetic field generation devices (3.0) which are located directly below the mounting device (1.2) (as shown for visualization purposes in Figs. 3A and 3B). Therefore, the magnetic field generation devices (3.0) are at a defined distance from the magnetic field generation device (3.0), herein in the range between 3 and 15 cm. The mounting device (1.2) has a substantially planar surface.

In this example the magnetic field generation devices (3.0) are electromagnetic coils, adapted to generate a transient magnetic field (4.0). The magnetic field generation devices (3.0) are arranged in a coil array, consisting for example of 46 magnetic field generation devices (3.0), arranged in 5 rows in a manner to provide a magnetic field to the whole target or individual parts. This corresponds to a magnetic field generation devices density of about 21 devices per square meter (m²) of the surface (1.2.1). The control and supply circuit (5.0), adapted to supply power to and/or control at least one parameter of the transient magnetic field (4.0), is configured, in this example, to individually provide an control each individual magnetic field generation device (3.0) in the array, therefore the magnetic field can be modulated and spatially arranged to match the specific anatomical proportions of the target (2.0). The control and supply circuit (5.0) includes, for each of the magnetic field generation devices (3.0), one or more input connectors (e.g., including a plug and/or, e.g., a cable lug) electrically coupled to the electromagnetic coil, such that the supply circuit is configured to supply each of the magnetic field generation devices (3.0) individually (selectively) with electrical power.

### EXAMPLE 2:

This example (see Figs. 3A to 3D for illustrative examples) is a specific embodiment of example 1, wherein the apparatus provides, in addition to the magnetic field generation devices (3.0) arranged in a coil array consisting of 46 magnetic field generation devices (3.0), two further magnetic field generation devices (3.1) arranged parallel to the target plane (2.7) or the longitudinal axis (2.7.1) (see Figs. 2A/2B for reference). The two further magnetic field generation devices (3.1) are adapted to generate a magnetic field parallel to the plane, which allows to modulate the magnetic field (4.0) and to provide another exposure vector and are designed as Helmholtz coils. This embodiment includes an actuator coupled to the support structure and to the further magnetic field generation device(s) (3.1) and configured to change the position of the further magnetic field generation devices (3.1) relative to the surface (1.2.1) of the mounting device (1.2). This allows a more complex field distribution.

### EXAMPLE 3:

In this exemplary embodiment of the apparatus (1.0) (see Fig. 3F for illustrative example), the mounting device (1.2) has a contoured (or "distorted") surface (1.2.1), here designed as a chair. This surface (1.2.1) is ergonomically optimized to conform to the shape of the body while maintaining a defined target position. In these cases, each magnetic field generation device (3.0) is arranged in a perpendicular or substantially perpendicular orientation relative to the local surface contour. The support structure (1.1) is integrated with the mounting device (1.2) to allow the field generation device(s) (3.0) to be positioned substantially perpendicular to the surface (1.2.1). Therefore, this embodiment can be interpreted as a distorted planar mounting device (3.0), as described in example 1 or 2, adapted to the surface of the body of the target, particularly a human body. This facilitates long time exposures.

### EXAMPLE 4:

In this specific embodiment of the apparatus of examples 1 to 3 (see for example, Fig.3A to Fig.3F) the distance of each of the multiple coils from the support surface is less than 0.25 m, e.g., less than 0.1 m, e.g., less than 0.05 m. This increases the magnetic field (e.g., the strength thereof) at the surface of the mounting device (1.2) and more importantly at the target comprised by the body (2.2) with a spatial volume.

### EXAMPLE 5:

This exemplary embodiment of the apparatus of one of examples 1 to 4, wherein each of the magnetic field generation devices (3.0), herein as electromagnetic coils has an inductance (also referred to as coil inductance) of less than 100 mH (e.g., less than 50 mH, e.g., less than 10 mH) and/or varies from a common reference inductance by less than 5 Millihenry (mH) and/or less than 5% of the common reference inductance, wherein, preferably, the reference inductance is between 0.5 and 50 mH (e.g., between 1 and 5 mH). This facilitates providing a transient magnetic field at repetition frequencies in the range between 1 Hz to 1000Hz, preferably between 5 Hz to 100 Hz, most preferably between 5 Hz and 50 Hz.

### EXAMPLE 6:

This embodiment of the invention (see, for example, Fig. 10A/10B) is a supply and control circuit (5.0), e.g., for supplying the device of one of the preceding examples with electrical power, the supply and control circuit (5.0) including: a capacitance circuit (5.1) (e.g., including one or more capacitors for) providing a capacitance; a power source circuit (5.2) (also referred to as power generation circuitry) configured to provide an electrical power; an operating circuit (5.3) including one or more output connectors (e.g., multiple output connectors for providing a multi-channel operation), e.g., configured as plug, for outputting the electrical power in accordance with multiple operation modes (for providing a multi-mode operation), of which: a pulse operation mode alternates a first phase and a second phase, wherein the capacitance circuit (5.1) is coupled to the power generation circuit (5.2) to charge the capacitance by the electrical power in the first phase, and wherein the capacitance circuit (5.1) is coupled to the one or more output connectors to discharge the capacitance via the one or more output connectors in the second phase; at least one continuous operation mode couples the power generation circuit (5.2) to the one or more output connectors to supply the electrical power to the one or more output connectors.

### EXAMPLE 7:

In this example (see, for reference, Fig. 10A) is the supply and control circuit (5.0) of example 6, wherein the operating circuit (5.3) includes an operation control input and one or more circuit configuration elements (e.g., switches) and is configured to change a state of the one or more circuit configuration element based on an operation control signal received at the operation control input; wherein preferably, each of the one or more circuit configuration element includes one or more than one electrical (e.g., bidirectional) relay. This facilitates the operation of the supply and control circuit (5.0) in the spirit of the current invention.

### EXAMPLE 8:

This example of the invention (see, for example, Fig. 10A) is the supply and control circuit (5.0) of example 6 or 7, wherein the power generation circuit (5.2) is configured to provide the electrical power via a direct current in the pulse operation mode (also referred to as first operation mode) and to provide the electrical power via an alternating current in the continuous operation mode (also referred to as second operation mode). This facilitates the operation of the supply and control circuit (5.0) in the spirit of the current invention.

### EXAMPLE 9:

This exemplary embodiment (see, for example, Fig. 10A) represents the supply and control circuit (5.0) of one of the examples 6 to 8, wherein the power generation circuit (5.2) includes a power control input and is configured to change the time dependency of a current providing the electrical power based on a power control signal received at the power control input. This facilitates the operation of the supply and control circuit (5.0) in the spirit of the current invention.

### EXAMPLE 10:

In this embodiment of (see, for example, Fig. 10A) of the supply and control circuit (5.0) of one of examples 6 to 9 the capacity circuit includes an operation control input and one or more (e.g., mechanical and/or electromechanical) switches and is configured to change a state of the one or more switches based on an operation control signal received at the operation control input; wherein preferably, the capacity of the capacity circuit is a function of the state of the one or more switches and/or wherein each of the one or more switches includes an electrical relay. This facilitates the operation of the supply and control circuit (5.0) in the spirit of the current invention.

### EXAMPLE 11:

This embodiment (see, for example, Fig. 10A) represents an example of the supply and control circuit (5.0) of one of examples 6 to 10, wherein the capacitance circuit (5.1) includes a capacitance control input, wherein the capacitance circuit (5.1) is configured to change the capacitance (e.g., being exposed to the electric power) based on a capacitance control signal received at the capacitance control input. This facilitates the operation of the supply and control circuit (5.0) in the spirit of the current invention.

### EXAMPLE 12:

This embodiment (see, for example, Fig. 10A) represents an example of the supply and control circuit (5.0) of one of examples 6 to 11, wherein the capacitance circuit (5.1) includes multiple sub-circuitries (also referred to as capacity group) coupled in parallel, of which each sub-circuitry includes a capacitor and a circuit configuration element (e.g., being addressable via the control input), e.g., switch. This facilitates the operation of the supply and control circuit (5.0) in the spirit of the current invention.

### EXAMPLE 13:

In this embodiment (see, for example, Fig. 10A) of the supply and control circuit (5.0) of one of examples 6 to 12 the operating circuit (5.3) includes an output control input, and is configured to configure (e.g., change) which of the connectors outputs the electrical power, based on an output control signal received at the output control input; wherein preferably, the multiple connectors include a set of first connectors, and for each first connector of the set of first connectors, a second connector, and wherein preferably the operating circuit (5.3) is configured to output the electrical power alternately via the first connector and the second connector. This facilitates the operation of the supply and control circuit (5.0) in the spirit of the current invention.

### EXAMPLE 14:

This embodiment (see, for example, Fig. 10A) represents an example of the supply and control circuit (5.0) of one of examples 6 to 13, wherein the multiple connectors include a first connector and a second connector, wherein the operating circuit (5.3) is configured such that the electrical power is output via the first connector and the second connector in a time-displaced manner, preferably wavelike. This allows for a more complex transient magnetic field distribution.

### EXAMPLE 15:

This embodiment (see, for example, Fig. 10A) represents an example of the supply and control circuit (5.0) of any one of examples 6 to 14, wherein the at least one continuous operation mode includes a first continuous operation mode and a second continuous operation mode, wherein in the first continuous operation mode (resonance mode) the capacitance circuit (5.1) is operated in resonance with an inductance coupled to the one or more output connectors; wherein in the second continuous operation mode (direct mode) the capacitance circuit (5.1) is galvanically isolated from the one or more output connectors. This allows for a more complex transient magnetic field distribution.

### EXAMPLE 16:

This example relates to an in vitro method (ex vivo) for enhancing neuronal cell activity, here enhancing neuronal activity in healthy human motor neuron cells (2.4) as a cell culture (6.0) and damaged motor neuron cells (2.4) from a human ALS patient as a cell culture (6.0) of mutant FUS-GFP P525L (ALS). In this example, the cells were cultured in a cell culture architecture as shown in Figure 9, with a distal axon compartment (2.5.7) and a proximal somatic site (2.5.8) connected by microgrooves (2.5.9). Here the cells are cultured for 30 days and then exposed to a transient magnetic field according to the method of the present invention. In this example, FUS-GFP WT MNs (Ctrl) were compared against isogenic mutant FUS-GFP P525L MNs (ALS), all transient magnetic field exposures (MFE) were performed at a flux density of 10 mT and the indicated repetition frequency (x-axis) and compared with unexposed cells (i.e., at 0 Hz) (see Figure 4). The entire pool of fluorescence microscopy image series (movies) examined was analyzed with respect to four key tracking parameters (y-axis): mean velocity in µm/s (Fig. 4A and 4B), track displacement in µm (Fig. 4C and 4D), moving fraction in % (Fig. 4E and 4F), and antero-/retrograde motility ratio (Fig. 4G and 4H) as a function of repetition frequency (x-axis) at a perpendicular orientation of the axonal plane relative to the magnetic field. Exposure was performed three times for 7 h every 5 days after cell maturation for 30 days (i.e., on day 30, 35, and 40) and finally once for 3.5 h on day 45. On day 48, live fluorescent video microscopy was performed to assess the axonal organelle motility. Data points are the mean values of the total organelle population analyzed, resulting in sample sizes typically in the range of tens of thousands across all pooled fluorescence microscopy image series (movies) per condition, i.e. for a given frequency and cell line, with error bars representing the standard deviation.

### REFERENCE LIST

| | |
|---|---|
| (1.0) | Apparatus for exposing a target to a magnetic field |
| (1.1) | Support structure |
| (1.2) | Mounting device |
| (1.2.1) | Surface of the mounting device |
| (2.0) | Target |
| (2.1) | Target location |
| (2.2) | Body |
| (2.3) | Neurons |
| (2.4) | Motor neurons |
| (2.4.1) | Upper motor neurons |
| (2.4.2) | Lower motor neurons |
| (2.4.3) | Muscle fibers |
| (2.5) | Axons |
| (2.5.1) | Microtubule |
| (2.5.2) | Mitochondrion |
| (2.5.3) | Kinesin |
| (2.5.4) | Lysosome |
| (2.5.5) | Cytoplasmic dynein |
| (2.5.6) | Dynactin |
| (2.5.7) | Distal axon compartment |
| (2.5.8) | Proximal somatic side |
| (2.5.9) | Microgroove |
| (2.6) | Mean axonal plane |
| (2.6.1) | Axonal axis |
| (3.1) | Further magnetic field generation device |
| (2.7) | Target (coronal) plane |
| (2.7.1) | Longitudinal (craniocaudal) axis |
| (3.0) | Magnetic field generation device |
| (3.1) | Further magnetic field generation device |
| (4.0) | Magnetic field |
| (4.1) | Oscillating field |
| (5.0) | Control and supply circuit |
| (5.1) | Capacitance circuit |
| (5.2) | Power generation circuit |
| (5.3) | Operating circuit |
| (5.4) | Pulse damping circuit |
| (5.5) | Pulse enable circuit |
| (6.0) | Cell culture |

## Claims

1. An **apparatus (1.0),** configured to expose a target (2.0) to a magnetic field (4.0), comprising:
- a support structure (1.1),
- a mounting device (1.2) configured to receive and/or support the target (2.0),
- at least one magnetic field generation device (3.0), adapted to generate a transient magnetic field (4.0),
- a control and supply circuit (5.0), adapted to supply power to and/or control at least one parameter of the transient magnetic field (4.0),
wherein the target (2.0) is positioned at a target location (2.1), the target location (2.1) being at a defined distance from the magnetic field generation device (3.0), and
wherein the target (2.0) is surrounded by a body (2.2) of a spatial volume, the body (2.2) having a specific magnetic permeability, and
wherein the target (2.0) comprises or consists of neurons (2.3), preferably motor neurons (2.4) with axons (2.5),
wherein the transient magnetic field (4.0) is a pulsed and/or oscillating magnetic field with a repetition frequency and a flux density,
**characterized in that:**
- the flux density of the magnetic field (4.0) is spatially adjustable, and
- the flux density of the magnetic field (4.0) at the target location (2.1) is between 5 mT and 100 mT, and
- the repetition frequency of the magnetic field (4.0) is between 1 Hz and 200 Hz.

2. The apparatus (1.0) according to claim 1, wherein the magnetic field is substantially perpendicular to the target (2.0).

3. The apparatus (1.0) according to any of the claims 1 or 2, wherein target (2.0) is preferably selected from the list consisting of isolated cells, cell clusters, cell cultures (6.0), microorganisms, tissue sections, or isolated organs, spine, head, peripheral body parts, in particular legs and/or arms, torso, whole body or a combination thereof.

4. The apparatus (1.0) according to any of the claims 1 to 3, wherein the flux density output as measured at the surface of the mounting device (1.2.1) is chosen in a way that the magnetic flux density is between 5 mT and 100 mT at the target location (2.1), wherein the target location (2.1) has an spatial elevation of 10 µm and 50 cm above the surface (1.2.1) and a specific magnetic permeability of 1.0×10⁻⁶ H/m to 1.5×10⁻⁶ H/m.

5. The apparatus (1.0) according to any of the claims 1 to 4, wherein the magnetic field (4.0) has a wave function selected from the list consisting of sinusoidal wave, square wave, bi- or multiphasic square wave.

6. The apparatus (1.0) according to any of the claims 1 to 5, wherein the apparatus (1.0) comprises an arrangement of 2 to 100 magnetic field generation devices (3.0) positioned substantially perpendicular in relation to the mean axonal plane (2.6) and/or the target plane (2.7), wherein substantially perpendicular preferably means that the magnetic field (4.0) is at an angle of 0 to 40° to the surface normal of the mean axonal plane (2.6) and/or to the surface normal of the target plane (2.7).

7. The apparatus (1.0) according to any of the claims 1 to 6, wherein the apparatus (1.0) comprises at least one further magnetic field generation device(s) (3.1), preferably arranged in parallel to the surface (1.2.1) of the mounting device (2.1), which is designed to envelop the entire body and/or an part of the body of the target.

8. The apparatus (1.0) according to any of the claims 1 to 7, wherein the magnetic field generation device(s) (3.0; 3.1) is/are selected from the list consisting of electromagnetic helix or spiral coils, also referred to as solenoid coils, air coils, solid core coils, Helmholtz coils, racetrack coils or a combination thereof.

9. The apparatus (1.0) according to any of the claims 1 to 8, wherein the target (2.0) comprises motor neurons (2.4), wherein the magnetic field (4.0) increases a neuronal cell activity, in particular axonal organelle transport activity, selected from the list consisting of mean velocity, track displacement, moving fraction, ratio of anterograde to retrograde tracks, by at least 20%, as determined by video fluorescence microscopy measurements.

10. The apparatus (1.0) according to any of the claims 1 to 9, wherein the target (2.0) is a body of a subject or a group of subjects suffering or at risk from suffering from a neurodegenerative disease, preferably a neurodegenerative disease selected from the list consisting of amyotrophic lateral sclerosis (ALS), primary lateral sclerosis (PLS), progressive muscular atrophy (PMA), spinal muscular atrophy (SMA), Alzheimer's, Parkinson's, Huntington's, Creutzfeldt-Jakob Disease (CJD) or diabetic polyneuropathy.

11. A **control and supply circuit** (5.0) according to any of the claims 1 to 10, comprising
- a capacitance circuit (5.1) for providing a capacitance,
- a power generation circuit (5.2) configured to provide electrical power; and
- an operating circuit (5.3) comprising one output terminals for outputting the electrical power in accordance with a plurality of operating modes, of which
wherein the operating modes comprises a pulsed mode of operation alternating between a first phase and a second phase,
wherein the capacitance circuit (5.1) is coupled to the power generation circuit (5.2) to charge the capacitance by the electrical power in the first phase, and wherein the capacitance circuit (5.1) is coupled to the one output connectors to discharge the capacitance via the one output connectors in the second phase,
**characterized by that**
- in at least one continuous operation mode, coupling the power generation circuit (5.2) to the one output connectors to supply the electrical power to the one output connectors, and
- the control and supply circuit (5.0) provides the electric power to generate the transient magnetic field (4.0) according to any of the claims 1, 2, 3 or 5.

12. The control and supply circuit according to claim 11, wherein the capacitance circuit (5.1) includes a capacitance control input, wherein the capacitance circuit (5.1) is configured to change the capacitance based on a capacitance control signal received at the capacitance control input; and/or wherein the capacitance circuit (5.1) includes multiple sub-circuitries coupled in parallel, of which each sub-circuitry includes a capacitor and a switch..

13. A **method** for enhancing neuronal activity in vitro using an apparatus according to any of the claims 1 to 10, the method comprising the following steps:
a) Providing a target (2.0) comprising or consisting of neurons, in particular motor neurons with axons,
b) Placing the target (2.0) on a mounting device, then
c) Exposing the target (2.0) to a pulsed and/or oscillating magnetic field during a fixed time interval, wherein the magnetic field has a flux density and a repetition frequency, wherein the flux density is between 5 mT and 100 mT and the repetition frequency is between 1 Hz and 200 Hz, then preferably rest the target (2.0) for a fixed time interval,
d) Repeat step c several times to reach a total procedure time of 10 min to 12 hours,
**characterized in that**
- the target (2.0) comprises, preferably is, an in vitro neuron, preferably an in vitro motor neuron,
- the target (2.0) is comprised in a volume, preferably between 1 µm³ and 0.5 m³,
- the volume has a spatial elevation above the surface (1.2.1), preferably between 10 µm and 50 cm,
- the volume has a specific permeability, preferably between 1.0×10⁻⁶ H/m to 1.5×10⁻⁶ H/m.

14. The method for increasing neuronal activity in vitro according to claim 13, wherein the target (2.0) comprises or consists of motor neurons (2.4) with axons (2.5), wherein the magnetic field (4.0) increases a neuronal cell activity, in particular axonal organelle transport activity, selected from the list consisting of mean velocity, track displacement, moving fraction, ratio of anterograde to retrograde tracks, by at least 20%, as determined by video fluorescence microscopy measurements.

15. The method for increasing neuronal activity in vitro according to claim 13, wherein the target (2.0) comprises or consists of motor neurons (2.4) with axons (2.5), wherein the magnetic field (4.0) increases the DNA repair activity of damaged cell nuclei by at least 20%, preferably by 20% to 300%, compared to targets not exposed to the field, as determined by fluorescence microscopy.

16. An **exposure plan** for exposing a target (2.0), preferably an in vitro target, comprising neurons to a magnetic field with an apparatus (1.0) according to any of the claims 1 to 10, with a method according to any of the claims 13 or 14, wherein the method is applied in fixed time intervals, wherein the fixed time interval is between 1 day and 2 weeks.
